# EUROPEAN PATENT SPECIFICATION

(11) **EP 1 539 723 B1**
(45) Date of publication and mention of the grant of the patent: **24.05.2006**
(21) Application number: 03788208.1
(22) Date of filing: 13.08.2003
(51) Int. Cl.: C07D 277/62, C07D 417/02, C07D 417/14, A61K 31/428, A61K 31/495, A61K 31/435, A61K 31/5377, A61P 11/06, A61P 37/08

(54) **NOVEL USE OF BENZOTHIAZOLE DERIVATIVES**
NEUE VERWENDUNG VON BENZOTHIAZOLDERIVATEN
NOUVELLE UTILISATION DE DERIVES DE BENZOTHIAZOLE

(30) Priority: 14.08.2002 SE 0202429
(43) Date of publication of application: 15.06.2005
(73) Proprietor: AstraZeneca AB, 151 85 Södertälje (SE)
(72) Inventor: SJÖ, Peter, S-221 87 Lund (SE); STORM, Peter, S-221 87 Lund (SE)
(86) International application number: PCT/SE2003/001271
(87) International publication number: WO 2004/016600

(56) References cited:
- EP-A1- 0 483 502
- WO-A1-99/24035
- WO-A1-02/051821
- WO-A2-01/74810
- DD-A- 150 059
- GB-A- 1 363 691
- GB-A- 1 363 692
- US-A- 3 257 204
- US-A- 5 552 421
- KLINE ET AL.: 'Antimicrobial Effects of Novel Siderophores Linked to Beta-Lactam Antibiotics' BIOORGANIC & MEDICINAL CHEMISTRY vol. 8, no. 1, 2000, pages 73 - 93, XP002204722
- STEVENS ET AL.: 'Structural Studies on Bioactive Compounds. 23. Synthesis of Polyhydroxylated 2-Phenylbenzothiazoles and a Comparison of Their Cytotoxicities and Pharmacological Properties with Genistein and Quercetin' J. MED. CHEM. vol. 37, no. 11, 1994, pages 1689 - 1695, XP002236361
- DATABASE CAPLUS [Online] OTSUKA PHARMACEUTICAL CO. LTD.: 'Preparation of Benzothiazoles and Benzimidazoles as blood platelet aggregation inhibitors', XP002974378 Retrieved from STN Database accession no. 1991:207259 & JP 20002306916 A 22 October 2002
- DATABASE CAPLUS [Online] SANTEN PHARMACEUTICAL CO. LTD.: 'Preparation of 2-phenylbenzothiazoline derivatives as cardiovascular agents', XP002974379 Retrieved from STN Database accession no. 1988:167460 & JP 62 221 679 A

## Description

This invention relates to the use of benzothiazole derivatives as inhibitors of the kinase Itk. Certain novel benzothiazole derivatives are also disclosed together with processes for their preparation, intermediates thereto, pharmaceutical compositions comprising them, and their use in therapy.

### Background of the Invention

Inducible T cell Kinase (Itk) is a member of the Tec-family of cytosolic protein tyrosine kinases. In mammalians, this family also includes Btk, Tec, Bmx, and Txk. These kinases regulate various immune cell functions that integrate signals given by the other cytosolic tyrosine kinases as well as serine/threonine kinases, lipid kinases, and small G proteins. Tec-family kinases have the following general structure: a N-terminal pleckstrin-homology (PH) domain, a Tec-homology domain that includes a Btk motif and one or two proline-rich (PR) motifs, a SH3 domain, a SH2 domain and a c-terminal catalytic (SH1) domain. These kinases are expressed exclusively in hematopoietic tissues, with the exception of Tec and Bmx that have also been detected in endothelial cells. The cellular distribution is different for the Tec-family members. For example, Itk is expressed by T cells, NK cells and mast cells, whereas Btk is expressed by all hematopoietic cells except T cells. Thus, hematopoietic cells may express one or several Tec-family kinases. For example, T cells express Itk, Tec and Txk, and mast cells express Btk, Itk and Tec.
Btk is by far the most extensively studied among the Tec-family kinases, due to its association with X-linked agammaglobulinemia (XLA), and Btk is currently the only Tec-family kinase with a known human phenotype. XLA patients are virtually devoid of mature B cells and their Ig levels are strongly reduced.
Itk^{-/-} mice show defects in T cell activation and differentiation. T helper 2 (Th2) differentiation is disrupted in these mice, whereas Th1 differentiation is apparently intact.

In T and B cells, signalling through T cell receptors and B cell receptors leads to activation of Itk and Btk, respectively. Downstream of Itk and Btk a number of different messengers are engaged; scaffolding proteins (SLP-76, LAT, SLP-65), Src kinases, MAP kinases, and PI3-K. These events are followed by PLC-γ activation that leads to IP3 generation and sustained Ca²⁺ flux, and subsequently activation of transcription factors. PLC-γ1 has been suggested as a direct substrate for Itk.
In T cells, Itk (and Tec) may also mediate signalling through the CD28 co-receptor. Furthermore, Itk has in T cells been implicated in the activation of β-integrin.
Signalling from Tec-family kinases can also be regulated by PH domain-mediated plasma membrane localization, and by Src-family-mediated phosphorylation of critical tyrosine residues. Interestingly, Itk, Btk and Txk have recently been shown to translocate to the nucleus after activation.

From studies using Itk-/- mice, it has been proposed that Itk is required for Th2 but not Th1 cell development. This was demonstrated in the *N. brasiliensis* and *L*. *major* infection models where the Itk-/- animals are protected in the Leishmania model indicating an intact Th1 response, whereas they are susceptible to infection with *N. Brasiliensis* that requires an intact Th2 response for resolution of the infection. This indicates that modulation of Itk activity may prove useful for treatment of Th2-driven disorders and conditions.

We have identified the critical role of Itk in regulating important mast cell and basophil functions and it is shown that the activity of mast cells or basophils may be inhibited through inhibition of Itk. Thus Itk inhibitors may be used as pharmaceutical agents for the treatment of mast cell-driven or basophil-driven conditions or diseases. In particular, we have identified Itk as a target for inhibiting several key events in both acute and late phase allergic reactions common to allergic rhinitis and asthma.

Certain 2-(2-hydroxyphenyl)benzothiazole derivatives have been previously disclosed in the literature but no such compounds have been disclosed to have utility as kinase inhibitors, particularly not as inhibitors of the kinase Itk.

The present invention discloses certain 2-aryl benzothiazole derivatives that have activity as Itk inhibitors and are thereby useful as pharmaceuticals.

### Disclosure of the Invention

The present invention provides the use of a compound of formula (I) 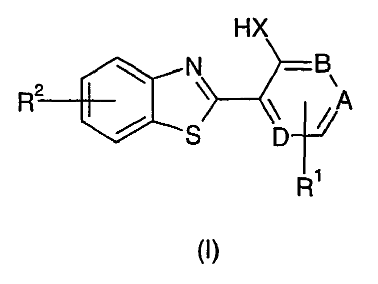 wherein:
A and D independently represent CH or N; and
either B represents CH and X represents O;
or B represents N and X represents NH;
R¹ represents hydrogen, halogen, C1 to 6 alkyl, C1 to 6 alkoxy, aryl or benzyloxy; said aryl or benzyloxy group being optionally further substituted by a group selected from C1 to 6 alkyl, C1 to 6 alkoxy, halogen and carbomethoxy;
R² represents hydroxy, amino, C1 to 6 alkyl, C1 to 6 alkoxy, carbamoyl (-CONR³R⁴) or -COOH; said alkyl or alkoxy group being optionally further substituted by one or more groups independently selected from hydroxy and NR⁵R⁶;
R³, R⁴, R⁵ and R⁶ each independently represent hydrogen or C1 to 6 alkyl; said alkyl group being optionally further substituted by one or more substituents selected independently from hydroxy, C1 to 6 alkoxy, NR⁷R⁸ and C1 to 6 alkoxycarbonyl;
or the group NR³R⁴ or the group NR⁵R⁶ may together represent a 5 to 7 membered azacyclic ring optionally incorporating one further heteroatom selected from O and NR⁹;
R⁷ and R⁸ independently represent hydrogen or C1 to 6 alkyl; or the group NR⁷R ⁸ together represents a 5 to 7 membered azacyclic ring optionally incorporating one further heteroatom selected from O and NR⁹;
R⁹ represents hydrogen or C1 to 6 alkyl; said alkyl group being optionally further substituted by C1 to 6 alkoxy;
or a pharmaceutically acceptable salts thereof, in the manufacture of a medicament for the treatment or prophylaxis of diseases or conditions in which inhibition of kinase Itk activity is beneficial.

The compounds of formula (I) may exist in enantiomeric forms. It is to be understood that all enantiomers, diastereomers, racemates and mixtures thereof are included within the scope of the invention.

Compounds of formula (I) may also exist in various tautomeric forms. All possible tautomeric forms and mixtures thereof are included within the scope of the invention.

In one embodiment, B represents CH and X represents O.

In another embodiment, A and D each represent CH.

In another embodiment, R¹ represents hydrogen, halogen or C1 to 6 alkoxy.

In another embodiment, R² represents -CONR³R⁴ or C1 to 6 alkyl substituted by NR⁵R⁶.

In one embodiment, the substituent R² is located at the 5- or 6-position of the benzothiazole ring system. In another embodiment, the substituent R² is located at the 6-position of the benzothiazole ring system.

In one embodiment, the invention provides the use of a compound of formula (I) wherein A and D each represent CH; B represents CH and X represents O; R¹ represents hydrogen, halogen or C1 to 6 alkoxy, R² represents -CONR³R⁴ or C1 to 6 alkyl substituted by NR⁵R⁶; and the substituent R² is located at the 5- or 6-position of the benzothiazole ring system.

The present invention specifically includes the use of the following compounds:
2-(4-chloro-2-hydroxyphenyl)-1,3-benzothiazole-6-carboxylic acid;
2-(2-hydroxy-6-methoxyphenyl)-1,3-benzothiazole-6-carboxylic acid;
2-(2-hydroxy-5-methoxyphenyl)-1,3-benzothiazole-6-carboxylic acid;
2-(4-chloro-2-hydroxyphenyl)-N-[2-(dimethylamino)ethyl]-N-methyl-1,3-benzothiazole-6-carboxamide;
5-chloro-2-[6-(pyrrolidin-1-ylcarbonyl)-1,3-benzothiazol-2-yl]phenol;
2-(4-chloro-2-hydroxyphenyl)-N-(3-morpholin-4-ylpropyl)-1,3-benzothiazole-6-carboxamide;
2-(4-chloro-2-hydroxyphenyl)-N-(2-pyrrolidin-1-ylethyl)-1,3-benzothiazole-6-carboxamide;
5-chloro-2-(6-{[4-(2-methoxyethyl)piperazin-1-yl]carbonyl}-1,3-benzothiazol-2-yl)phenol;
2-(2-hydroxy-5-methoxyphenyl)-N-(3-morpholin-4-ylpropyl)-1,3-benzothiazole-6-carboxamide;
4-methoxy-2-[6-(pyrrolidin-1-ylcarbonyl)-1,3-benzothiazol-2-yl]phenol;
2-(5-bromo-2-hydroxyphenyl)-N-[2-(dimethylamino)ethyl]-N-methyl-1,3-benzothiazole-6-carboxamide;
4-fluoro-2-[6-(pyrrolidin-1-ylcarbonyl)-1,3 -benzothiazol-2-yl]phenol;
5-methoxy-2-[6-(pyrrolidin-1-ylcarbonyl)-1,3-benzothiazol-2-yl]phenol;
methyl N- {[2-(2-hydroxyphenyl)-1,3-benzothiazol-6-yl]carbonyl} serinate;
2-[6-(pyrrolidin-1-ylcarbonyl)-1,3-benzothiazol-2-yl]phenol;
2-(5-chloro-2-hydroxyphenyl)-N-(3-morpholin-4-ylpropyl)-1, 3-benzothiazole-6-carboxamide;
3-methoxy-2-[6-(pyrrolidin-1-ylcarbonyl)-1,3-benzothiazol-2-yl]phenol;
N,N-diethyl-2-(2-hydroxy-4-methoxyphenyl)-1,3-benzothiazole-6-carboxamide;
N-[2-hydroxy-1-(hydroxymethyl)ethyl]-2-(2-hydroxy-4-methoxyphenyl)-1,3-benzothiazole-6-carboxamide;
2-[6-(pyrrolidin-1-ylcarbonyl)-1,3-benzothiazol-2-yl]pyridin-3-ol;
methyl 4-{[2-(6-{[[2-(dimethylamino)ethyl](methyl)amino]carbonyl}-1,3-benzothiazol-2-yl)-3-hydroxyphenoxy]methyl}benzoate;
5-ethoxy-4-[6-(pyrrolidin-1-ylcarbonyl)-1,3-benzothiazol-2-yl]pyridin-3-ol;
N-[2-(dimethylamino)ethyl]-2-(4-hydroxy-1,1'-biphenyl-3-yl)-N-methyl-1,3-benzothiazole-6-carboxamide;
N-[2-(dimethylamino)ethyl]-2-(4-hydroxy-3'-methoxy-1,1'-biphenyl-3-yl)-N-methyl-1,3-benzothiazole-6-carboxamide;
5-chloro-2-(6-{[[2-(dimethylamino)ethyl](methyl)amino]methyl}-1,3-benzothiazol-2-yl)phenol;
5-chloro-2-(6-{[4-(2-methoxyethyl)piperazin-1-yl]methyl}-1,3-benzothiazol-2-yl)phenol;
5-chloro-2-[6-(pyrrolidin-1-ylmethyl)-1,3-benzothiazol-2-yl]phenol ;
4-fluoro-2-[6-(pyrrolidin-1-ylmethyl)-1,3-benzothiazol-2-yl]phenol;
2-[6-(pyrrolidin-1-ylmethyl)-1,3-benzothiazol-2-yl]phenol;
3-methoxy-2-[6-(pyrrolidin-1-ylmethyl)-1,3-benzothiazol-2-yl]phenol;
4-methoxy-2-[6-(pyrrolidin-1-ylmethyl)-1,3-benzothiazol-2-yl]phenol;
4-bromo-2-[6-(pyrrolidin-1-ylmethyl)-1,3-benzothiazol-2-yl]phenol;
5-methoxy-2-[6-(pyrrolidin-1-ylmethyl)-1,3-benzothiazol-2-yl]phenol;
4-chloro-2-[6-(pyrrolidin-1-ylmethyl)-1,3-benzothiazol-2-yl]phenol;
2-{ 6-[(diethylamino)methyl]-1,3-benzothiazol-2-yl -5-methoxyphenol;
5-ethoxy-4-[6-(pyrrolidin-1-ylmethyl)-1,3-benzothiazol-2-yl]pyridin-3-ol;
4-chloro-2-(6-{[(3-morpholin-4-ylpropyl)amino]methyl}-1,3-benzothiazol-2-yl)phenol;
2-[6-(pyrrolidin-I-ylmethyl)-1,3-benzothiazol-2-yl]pyridin-3-ol;
2-[5-(pyrrolidin-1-ylmethyl)-1,3-benzothiazol-2-yl]phenol;
5-chloro-2-[6-(piperidin-1-ylmethyl)-1,3-benzothiazol-2-yl]phenol;
5-chloro-2-[6-(4-methylpiperazin-1-ylmethyl)-1,3-benzothiazol-2-yl]phenol;
5-chloro-2-(6-[(diethylamino)methyl]-1,3-benzothiazol-2-yl) phenol;
5-chloro-2-{ 6-[(dimethylamino)methyl]-1,3-benzothiazol-2-yl }phenol;
2-[6-(hydroxymethyl)-1,3-benzothiazol-2-yl]phenol;
2-(6-amino-1,3-benzothiazol-2-yl)-4-methoxyphenol;
*N,N-*dimethyl-2-(2-hydroxy-4-methoxyphenyl)-1,3-benzothiazole-6-carboxamide;
*N*,*N*-dimethyl-2-(2-hydroxyphenyl)-1,3-benzothiazole-6-carboxamide;
3-[6-(pyrrolidin-1-ylcarbonyl)-1,3-benzothiazol-2-yl]pyrazin-2-amine;
2-(4-chloro-2-hydroxyphenyl)-1,3-benzothiazol-6-ol;
5-chloro-2-[6-(2-hydroxy-3-pyrrolidin-1-yl-propoxy)-1,3-benzothiazol-2-yl]-phenol;
5-chloro-2-[5-(pyrrolidin-1-ylcarbonyl)-1,3-benzothiazol-2-yl]phenol;
2-[5-(pyrrolidin-1-ylcarbonyl)-1,3-benzothiazol-2-yl]phenol;
*N*,*N*-dimethyl-2-(2-hydroxyphenyl)-1,3-benzothiazole-5-carboxamide;
5-chloro-2-[5-(pyrrolidin-1-ylmethyl)-1,3-benzothiazol-2-yl]phenol;
2-{5-[(dimethylamino)methyl]-1,3-benzothiazol-2-yl } phenol;
2-(2-hydroxyphenyl)-N-(3-morpholin-4-ylpropyl)-1,3-benzothiazole-7-carboxamide;
and pharmaceutically acceptable salts thereof.

Unless otherwise indicated, the term "C1 to 6 alkyl" referred to herein denotes a straight or branched chain alkyl group having from 1 to 6 carbon atoms. Examples of such groups include methyl, ethyl, n-propyl, i-propyl, n-butyl, i-butyl, t-butyl, pentyl and hexyl.

Unless otherwise indicated, the term "C1 to 6 alkoxy " referred to herein denotes an oxygen substituent bonded to a straight or branched chain alkyl group having from 1 to 6 carbon atoms. Examples of such groups include methoxy, ethoxy, n-propoxy, i-propoxy, n-butoxy, i-butoxy and s-butoxy.

Unless otherwise indicated, the term "halogen" referred to herein denotes fluorine, chlorine, bromine and iodine.

Unless otherwise indicated, the term "aryl" referred to herein denotes a C6 to 10 carbocyclic aromatic ring system. Examples include phenyl, naphthyl and indanyl.

Examples of a 5 to 7 membered azacyclic ring optionally incorporating one further heteroatom selected from O and NR⁹ include pyrrolidine, piperidine, morpholine and piperazine

The present invention includes compounds of formula (I) in the form of salts, in particular acid addition salts. Suitable salts include those formed with both organic and inorganic acids. Such acid addition salts will normally be pharmaceutically acceptable although salts of non-pharmaceutically acceptable acids may be of utility in the preparation and purification of the compound in question. Thus, preferred salts include those formed from hydrochloric, hydrobromic, sulphuric, phosphoric, citric, tartaric, lactic, pyruvic, acetic, succinic, fumaric, maleic, methanesulphonic and benzenesulphonic acids.

A more particular aspect of the invention provides the use of a compound of formula (I), or a pharmaceutically acceptable salt thereof, in the manufacture of a medicament for the treatment or prophylaxis of allergic, autoimmune, inflammatory, proliferative and hyperproliferative diseases and immune-mediated diseases including rejection of transplanted organs or tissues and Acquired Immunodeficiency Syndrome (AIDS).

According to the invention there is also provided a method of treating, or reducing the risk of, diseases or conditions in which inhibition of kinase Itk activity is beneficial, which comprises administering to a person suffering from or at risk of said disease or condition, a therapeutically effective amount of a compound of formula (I) or a pharmaceutically acceptable salt thereof.

More particularly, there is also provided a method of treating, or reducing the risk of allergic, autoimmune, inflammatory, proliferative and hyperproliferative diseases and immune-mediated diseases including rejection of transplanted organs or tissues and Acquired Immunodeficiency Syndrome (AIDS), which comprises administering to a person suffering from or at risk of said disease or condition, a therapeutically effective amount of a compound of formula (I) or a pharmaceutically acceptable salt thereof.

Examples of these conditions are:
(1) (the respiratory tract) airways diseases including chronic obstructive pulmonary disease (COPD) such as irreversible COPD; asthma, such as bronchial, allergic, intrinsic, extrinsic and dust asthma, particularly chronic or inveterate asthma (for example, late asthma and airways hyper-responsiveness); bronchitis; acute, allergic, atrophic rhinitis and chronic rhinitis including rhinitis caseosa, hypertrophic rhinitis, rhinitis purulenta, rhinitis sicca and rhinitis medicamentosa; membranous rhinitis including croupous, fibrinous and pseudomembranous rhinitis and scrofoulous rhinitis; seasonal rhinitis including rhinitis nervosa (hay fever) and vasomotor rhinitis; sarcoidosis, farmer's lung and related diseases, fibroid lung and idiopathic interstitial pneumonia; sinusitis, chronic rhinosinusitis, nasosinusal polyposis; pulmonary fibrosis;
(2) **(bone and joints)** rheumatoid arthritis, seronegative spondyloarthropathies (including ankylosing spondylitis, psoriatic arthritis and Reiter's disease), Behcet's disease, Sjogren's syndrome and systemic sclerosis;
(3) (skin) psoriasis, atopical dermatitis, contact dermatitis and other eczmatous dermitides, seborrhoetic dermatitis, Lichen planus, Pemphigus, bullous Pemphigus, Epidermolysis bullosa, urticaria, angiodermas, vasculitides, erythemas, cutaneous eosinophilias, uveitis, Alopecia areata and vernal conjunctivitis;
(4) (gastrointestinal tract) Coeliac disease, proctitis, eosinopilic gastro-enteritis, mastocytosis, Crohn's disease, ulcerative colitis, food-related allergies which have effects remote from the gut, for example, migraine, rhinitis and eczema;
(5) (other tissues and systemic disease) multiple sclerosis, atherosclerosis, Acquired Immunodeficiency Syndrome (AIDS), lupus erythematosus, systemic lupus, erythematosus, Hashimoto's thyroiditis, myasthenia gravis, type I diabetes, nephrotic syndrome, eosinophilia fascitis, hyper IgE syndrome, lepromatous leprosy, sezary syndrome and idiopathic thrombocytopenia pupura; tuberculosis;
(6) (allograft rejection) acute and chronic following, for example, transplantation of kidney, heart, liver, lung, bone marrow, skin and cornea; and chronic graft versus host disease.

We are particularly interested in Th2-driven and/or mast cell-driven and/or basophil-driven conditions or diseases.

Thus, a more particular aspect of the invention provides the use of a compound of formula (I) or a pharmaceutically acceptable salt thereof, in the manufacture of a medicament for the treatment or prophylaxis of Th2-driven and/or mast cell-driven and/or basophil driven diseases or conditions; and a method of treating, or reducing the risk of, Th2-driven and/or mast cell-driven and/or basophil driven diseases or conditions which comprises administering to a person suffering from or at risk of, said disease or condition, a therapeutically effective amount of a compound of formula (I) or a pharmaceutically acceptable salt thereof.

In a preferred aspect of the invention, we provide a method for the treatment or prevention of a reversible obstructive airway disease, especially asthma, which comprises administering a therapeutically effective amount of a compound of formula (I) or a pharmaceutically acceptable salt thereof to a human that is suffering from or susceptible to the disease. We also provide the use of a compound of formula (I) or a pharmaceutically acceptable salt thereof in the manufacture of a medicament for the treatment or prevention of a reversible obstructive airway disease, especially asthma.

In another preferred aspect of the invention, we provide a method for the treatment or prevention of rhinitis which comprises administering a therapeutically effective amount of a compound of formula (I) or a pharmaceutically acceptable salt thereof to a human that is suffering from or susceptible to rhinitis, especially allergic rhinitis. We also provide the use of a compound of formula (I) or a pharmaceutically acceptable salt thereof in the manufacture of a medicament for the treatment or prevention of rhinitis, especially allergic rhinitis.

Prophylaxis is expected to be particularly relevant to the treatment of persons who have suffered a previous episode of, or are otherwise considered to be at increased risk of, the disease or condition in question. Persons at risk of developing a particular disease or condition generally include those having a family history of the disease or condition, or those who have been identified by genetic testing or screening to be particularly susceptible to developing the disease or condition.

For the above mentioned therapeutic indications, the dose of the compound to be administered will depend on the compound employed, the disease being treated, the mode of administration, the age, weight and sex of the patient. Such factors may be determined by the attending physician. However, in general, satisfactory results are obtained when the compounds are administered to a human at a daily dosage of between 0.1 mg/kg to 100 mg/kg (measured as the active ingredient).

The compounds of formula (I) may be used on their own, or in the form of appropriate pharmaceutical formulations comprising the compound of the invention in combination with a pharmaceutically acceptable diluent, adjuvant or carrier. Particularly preferred are compositions not containing material capable of causing an adverse reaction, for example, an allergic reaction. Conventional procedures for the selection and preparation of suitable pharmaceutical formulations are described in, for example, "Pharmaceuticals - The Science of Dosage Form Designs", M. E. Aulton, Churchill Livingstone, 1988.

In another aspect the invention provides a pharmaceutical formulation comprising a therapeutically effective amount of a compound of formula (I), or a pharmaceutically acceptable salt thereof, in admixture with a pharmaceutically acceptable adjuvant, diluent or carrier, for use in the treatment or prophylaxis of diseases or conditions in which inhibition of kinase Itk activity is beneficial.

In a more particular aspect, the invention provides a pharmaceutical formulation comprising a therapeutically effective amount of a compound of formula (I), or a pharmaceutically acceptable salt thereof, in admixture with a pharmaceutically acceptable adjuvant, diluent or carrier, for use in the treatment or prophylaxis of allergic, autoimmune, inflammatory, proliferative and hyperproliferative diseases and immune-mediated diseases including rejection of transplanted organs or tissues and Acquired Immunodeficiency Syndrome (AIDS).

According to the invention, there is provided a pharmaceutical formulation comprising preferably less than 95% by weight and more preferably less than 50% by weight of a compound of formula (I) in admixture with a pharmaceutically acceptable diluent or carrier.

We also provide a method of preparation of such pharmaceutical formulations that comprises mixing the ingredients.

The compounds may be administered topically, for example, to the lungs and/or the airways, in the form of solutions, suspensions, HFA aerosols or dry powder formulations, for example, formulations in the inhaler device known as the Turbuhaler® ; or systemically, for example, by oral administration in the form of tablets, pills, capsules, syrups, powders or granules; or by parenteral administration, for example, in the form of sterile parenteral solutions or suspensions; or by rectal administration, for example, in the form of suppositories.

Dry powder formulations and pressurized HFA aerosols of the compounds of the invention may be administered by oral or nasal inhalation. For inhalation, the compound is desirably finely divided. The finely divided compound preferably has a mass median diameter of less than 10 µm, and may be suspended in a propellant mixture with the assistance of a dispersant, such as a C₈-C₂₀ fatty acid or salt thereof, (for example, oleic acid), a bile salt, a phospholipid, an alkyl saccharide, a perfluorinated or polyethoxylated surfactant, or other pharmaceutically acceptable dispersant.

The compounds of the invention may also be administered by means of a dry powder inhaler. The inhaler may be a single or a multi dose inhaler, and may be a breath actuated dry powder inhaler.

One possibility is to mix the finely divided compound with a carrier substance, for example, a mono-, di- or polysaccharide, a sugar alcohol, or an other polyol. Suitable carriers are sugars, for example, lactose, glucose, raffinose, melezitose, lactitol, maltitol, trehalose, sucrose, mannitol; and starch. Alternatively the finely divided compound may be coated by another substance. The powder mixture may also be dispensed into hard gelatine capsules, each containing the desired dose of the active compound.

Another possibility is to process the finely divided powder into spheres which break up during the inhalation procedure. This spheronized powder may be filled into the drug reservoir of a multidose inhaler, for example, that known as the Turbuhaler® in which a dosing unit meters the desired dose which is then inhaled by the patient. With this system the active compound, with or without a carrier substance, is delivered to the patient.

For oral administration the active compound may be admixed with an adjuvant or a carrier, for example, lactose, saccharose, sorbitol, mannitol; a starch, for example, potato starch, corn starch or amylopectin; a cellulose derivative; a binder, for example, gelatine or polyvinylpyrrolidone; and/or a lubricant, for example, magnesium stearate, calcium stearate, polyethylene glycol, a wax, paraffin, and the like, and then compressed into tablets. If coated tablets are required, the cores, prepared as described above, may be coated with a concentrated sugar solution which may contain, for example, gum arabic, gelatine, talcum, titanium dioxide, and the like. Alternatively, the tablet may be coated with a suitable polymer dissolved in a readily volatile organic solvent.

For the preparation of soft gelatine capsules, the compound may be admixed with, for example, a vegetable oil or polyethylene glycol. Hard gelatine capsules may contain granules of the compound using either the above mentioned excipients for tablets. Also liquid or semisolid formulations of the drug may be filled into hard gelatine capsules.

Liquid preparations for oral application may be in the form of syrups or suspensions, for example, solutions containing the compound, the balance being sugar and a mixture of ethanol, water, glycerol and propylene glycol. Optionally such liquid preparations may contain colouring agents, flavouring agents, saccharine and/or carboxymethylcellulose as a thickening agent or other excipients known to those skilled in art.

The compounds of the invention may also be administered in conjunction with other compounds used for the treatment of the above conditions.

Certain compounds of formula (I) are novel.

Therefore a further aspect of the invention provides a compound of formula (Ia): 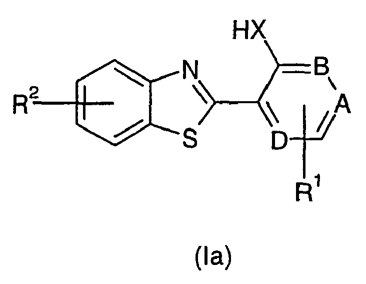 wherein:
A and D independently represent CH or N; and
either B represents CH and X represents O;
or B represents N and X represents NH;
R¹ represents hydrogen, halogen, C1 to 6 alkyl, C1 to 6 alkoxy, aryl or benzyloxy; said aryl or benzyloxy group being optionally further substituted by a group selected from C1 to 6 alkyl, C1 to 6 alkoxy, halogen and carbomethoxy;
R², which is attached to the 5- or 6-position of the benzothiazole ring, represents CONR³R⁴ or C1 to 6 alkyl or C1 to 6 alkoxy; said alkyl or alkoxy group being further substituted by one or more groups independently selected from hydroxy and NR⁵R⁶;
NR³ R⁴ represents a 5 to 7 membered azacyclic ring optionally incorporating one further heteroatom selected from O and NR⁹;
R⁵ and R⁶ each independently represent hydrogen or C1 to 6 alkyl; said alkyl group being optionally further substituted by one or more substituents selected independently from hydroxy, C1 to 6 alkoxy, NR⁷R⁸ and C1 to 6 alkoxycarbonyl;
or the group NR⁵R⁶ together represents a 5 to 7 membered azacyclic ring optionally incorporating one further heteroatom selected from O and NR⁹;
R⁷ and R⁸ independently represent hydrogen or C1 to 6 alkyl; or the group NR⁷R⁸ together represents a 5 to 7 membered azacyclic ring optionally incorporating one further heteroatom selected from O and NR⁹;
R⁹ represents hydrogen or C1 to 6 alkyl; said alkyl group being optionally further substituted by C1 to 6 alkoxy;
or a pharmaceutically acceptable salts thereof.

In one embodiment, B in formula (Ia) represents CH and X represents O.

In another embodiment, A and D in formula (Ia) each represent CH.

In another embodiment, R¹ in formula (Ia) represents hydrogen, halogen or C1 to 6 alkoxy.

In another embodiment, R² in formula (Ia) represents -CONR³R⁴ or C1 to 6 alkyl substituted by NR⁵R⁶.

In another embodiment, the substituent R² in formula (Ia) is located at the 6-position of the benzothiazole ring system.

In one embodiment, the invention provides a compound of formula (Ia) wherein A and D each represent CH; B represents CH and X represents O; R¹ represents hydrogen, halogen or C1 to 6 alkoxy, R represents -CONR³R⁴ or C1 to 6 alkyl substituted by NR⁵R⁶; and the substituent R² is located at the 6-position of the benzothiazole ring system.

Particular novel compounds of formula (1a) include those described within the Examples section of the present specification, either as such, or as the corresponding free bases, or as pharmaceutically acceptable salts thereof.

According to the invention there is also provided a compound of formula (Ia) or a pharmaceutically acceptable salt thereof for use as a medicament.

In a further aspect the invention provides a process for the preparation of a compound of formula (Ia) which comprises:
(a) reaction of a compound of formula (II) 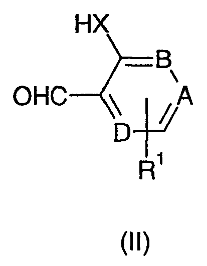 wherein R¹, A, B, D and X are as defined in formula (Ia), with a compound of formula (III) 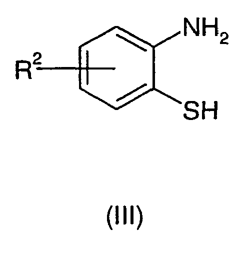 wherein R² is as defined in formula (Ia); or
(b) oxidative cyclisation of a compound of formula (IV) 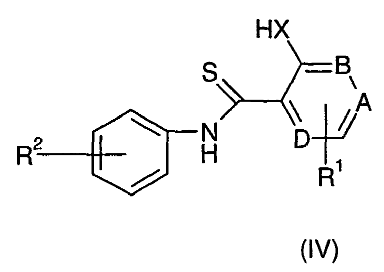 wherein R¹, R², A, B, D and X are as defined in formula (Ia);
and where desired or necessary converting the resultant compound of formula (Ia), or another salt thereof, into a pharmaceutically acceptable salt thereof; or converting one compound of formula (Ia) into another compound of formula (Ia); and where desired converting the resultant compound of formula (Ia) into an optical isomer thereof.

In process (a), the reaction may be achieved by heating together a mixture of compounds (II) and (III) in an oxidizing solvent, for example, nitrobenzene, at an elevated temperature, for example, at about 185 °C.

In process (b), the reaction may be achieved by suspending the compound of formula (IV) in aqueous base and then heating it together with a suitable oxidising agent such as potassium ferricyanide.

Compounds of formula (Ia) in which R² represents -CONR³R⁴ may be prepared by reacting a compound of formula (V) 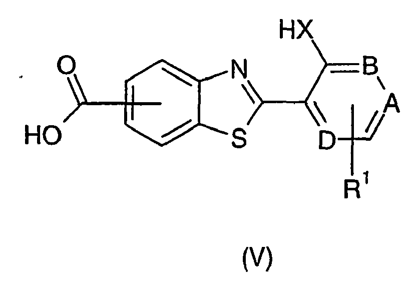 wherein R¹, A, B, D and X are as defined in formula (Ia), with an amine of the general formula (VI)

HNR³R⁴ (VI)

in which R³ and R⁴ are as defined in formula (Ia).

Methods that are suitable for the reaction of a compound of formula (V) with a compound of general formula (VI) will in general be well known to the man skilled in the art. For example, the two compounds may be coupled together in the presence of a coupling agent, for example, HATU.

Compounds of formula (Ia) in which R² represents -CH₂NR⁵R⁶ may be prepared by reduction of corresponding compounds in which R² represents -CONR⁵R⁶ with a reducing agent such as, for example, lithium aluminium hydride.

Compounds of formula (IV) may be prepared from corresponding compounds of formula (VI): 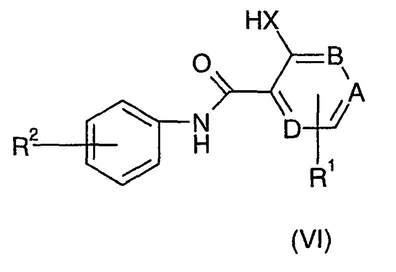 wherein R¹, R², A, B, D and X are as defined in formula (Ia), with a sulphurising agent such as Lawesson's reagent or phosphorus pentasulphide under conditions that will be readily apparent to the man skilled in the art.

Compounds of formulae (II), (III), (V) and (VI) are either commercially available, or are previously described in the chemical literature, or may be prepared using standard methods that are well known in the art.

Salts of compounds of formula (Ia) may be formed by reacting the free base or a salt, enantiomer, tautomer or protected derivative thereof, with one or more equivalents of the appropriate acid. The reaction may be carried out in a solvent or medium in which the salt is insoluble, or in a solvent in which the salt is soluble followed by subsequent removal of the solvent *in vacuo* or by freeze drying. Suitable solvents include, for example, water, dioxan, ethanol, 2-propanol, tetrahydrofuran or diethyl ether, or mixtures thereof. The reaction may be a metathetical process or it may be carried out on an ion exchange resin.

Compounds of formula (Ia) and intermediate compounds thereto may be prepared as such or in protected form. The protection and deprotection of functional groups is, for example, described in 'Protective Groups in Organic Chemistry', edited by J. W. F. McOmie, Plenum Press (1973), and 'Protective Groups in Organic Synthesis', 3rd edition, T. W. Greene & P. G. M. Wuts, Wiley-Interscience (1999).

The compounds of the invention and intermediates may be isolated from their reaction mixtures, and if necessary further purified, by using standard techniques.

The compounds of formula (Ia) may exist in enantiomeric or diastereoisomeric forms or mixtures thereof, all of which are included within the scope of the invention. The various optical isomers may be isolated by separation of a racemic mixture of the compounds using conventional techniques, for example, fractional crystallisation or HPLC. Alternatively, the individual enantiomers may be made by reaction of the appropriate optically active starting materials under reaction conditions that will not cause racemisation.

Intermediate compounds may also exist in enantiomeric forms and may be used as purified enantiomers, diastereomers, racemates or mixtures thereof.

According to a further aspect of the invention we provide a compound of formula (Ia) or a pharmaceutically acceptable salt thereof, for use as a medicament.

The compounds of formula (Ia), and their pharmaceutically acceptable salts, are useful because they possess pharmacological activity in animals. The compounds of formula (Ia) have activity as pharmaceuticals, in particular as modulators of kinase activity, especially Itk kinase activity, and as such are predicted to be useful in therapy. They may be used in the treatment or prophylaxis of allergic, autoimmune, inflammatory, proliferative and hyperproliferative diseases and immune-mediated diseases including rejection of transplanted organs or tissues and Acquired Immunodeficiency Syndrome (AIDS).

Examples of these medical conditions are disclosed above.

The compounds of formula (Ia) may be used on their own, or in the form of appropriate pharmaceutical formulations comprising the compound of formula (Ia) in admixture with a pharmaceutically acceptable diluent, adjuvant or carrier. Particularly preferred are compositions not containing material capable of causing an adverse reaction, for example, an allergic reaction. Conventional procedures for the selection and preparation of suitable pharmaceutical formulations are described in, for example, "Pharmaceuticals - The Science of Dosage Form Designs", M. E. Aulton, Churchill Livingstone, 1988.

In another aspect the invention provides a pharmaceutical formulation comprising a therapeutically effective amount of a compound of formula (Ia) or a pharmaceutically acceptable salt thereof, in admixture with a pharmaceutically acceptable adjuvant, diluent or carrier.

The following Examples are intended to illustrate, but in no way limit the scope of the invention.

### General methods

All reactions were performed in dried glassware in an argon atmosphere at room temperature, unless otherwise noted. All reagents and solvents were used as received. Merck Silica gel 60 (0.040-0.063 mm) was used for preparative silica gel chromatography. A Kromasil KR-100-5-C18 column (250 x 20 mm, Akzo Nobel) and mixtures of acetonitrile/water at a flow rate of 10 ml/min were used for preparative HPLC. Reactions were monitored at 254 nm by analytical HPLC, using a Kromasil C-18 column (150 x 4.6 mm) and a gradient (containing 0.1 % trifluoroacetic acid) of 5 to 100% of acetonitrile in water at a flow rate of 1 ml/min. Evaporations of solvents were performed under reduced pressure using a rotary evaporator at a maximum temperature of 60 °C. Products were dried under reduced pressure at about 40 °C.
¹H-NMR spectra were recorded on a Varian Inova 400 MHz or Varian Mercury 300 MHz instrument. The central solvent peak of chloroform-*d* (δ_{H} 7.27 ppm), dimethylsulfoxide-*d*₆ (δ_{H} 2.50 ppm) or methanol-*d*₄ (δ_{H} 3.35 ppm) were used as internal references. Low resolution mass spectra obtained on a Hewlett Packard 1100 LC-MS system equipped with a APCI ionisation chamber.

### General procedure for the preparation of 2-arylbenzothiazole-6-carboxylic acids

### (Method 1)

A mixture of 4-amino-3-mercaptobenzoic acid (J. Med. Chem. **1997**, *40*, 105) (5 mmol) and an aromatic aldehyde (5.1 mmol) were dissolved in acetic acid (5 ml) and nitrobenzene (15 ml). The solution was heated at 185 °C for 15 minutes. After cooling the reaction mixture was made alkaline by the addition of 1M sodium hydroxide solution and extracted with ethyl acetate. Following acidification with conc. hydrochloric acid, the water phase was extracted with ethyl acetate. Drying and evaporation of the solvent gave the title compound.

### General procedure for preparation of 2-arylbenzothiazoles (Method 2)

***N-Aryl-thiobenzamides* :** The N-aryl-benzamide, which was synthesised by known methods (see, for example, J. Med. Chem. **1999**, 42, 4172) (53.6 mmol) was dissolved in dry toluene (200 mL). Lawesson's reagent (32.2 mmol) was added and the reaction mixture was heated to 80 °C for 1 h under an inert atmosphere. TLC (toluene : ethyl acetate, 2: 1) showed a distinct yellow spot from the product. The reaction mixture was evaporated to dryness and purified via column chromatography (toluene : ethyl acetate, 9: 1) and used directly in the next step.

***2-Arylbenzotlaiazoles*** : N-Aryl-thiobenzamide (40 mmol) was suspended in 1.5M sodium hydroxide solution (270 mL, 400 mmol). The suspension was slowly added (30 minutes) to a warm (80 °C) 1.8M aqueous solution of K₃[Fe(CN)₆] (160 mmol). The reaction mixture was stirred for another 30 minutes before it was diluted with water (500 mL) and extracted with ethyl acetate. After evaporation of the solvent, the crude product was purified by column chromatography (toluene : ethyl acetate, 10:1) and recrystallised.

Following the protocol for the preparation of 2-arylbenzothiazole-6-carboxylic acids (Method 1), the following compounds (Examples 1 to 3) were synthesised:

### Example 1 2-(4-Chloro-2-hydroxyphenyl)-1,3-benzothiazole-6-carboxylic acid

The title compound was isolated in 44% yield.
¹H-NMR (400 MHz, DMSO-d₆): δ 12.00 (1H, brs); 8.76 (1H, s); 8.32 (1H, d); 8.11-8.05 (2H, m); 7.16 (1H, d); 7.09 (1H, dd).
APCI-MS m/z: 306 [MH⁺].

### Example 2 2-(2-Hydroxy-6-methoxyphenyl)-1,3-benzothiazole-6-carboxylic acid

The title compound was isolated in 44% yield.
¹H-NMR (400 MHz, DMSO-d₆): δ 8.78 (1H, s); 8.18-8.08 (2H, m); 7.45 (1H, t); 6.77 (1H, d); 6.73 (1H, d).
APCI-MS m/z: 302.1 [MH⁺].

### Example 3 2-(2-Hydroxy-5-methoxyphenyl)-1,3-benzothiazole-6-carboxylic acid

The title compound was isolated in 64% yield.
¹H-NMR (400 MHz, DMSO-d₆): δ 11.16 (1H, brs); 8.74 (1H, s); 8.12-8.04 (2H, m); 7.80 (1H, d); 7.11-7.04 (2H, m); 3.80 (3H, s).
APCI-MS m/z: 302.1 [MH⁺].

### General procedure for the amidation of 2-arylbenzothiazole-6-carboxylic acids

A mixture of 2-arylbenzothiazole-6-carboxylic acid (1 mmol), HATU (1.2 mmol) and *N*,*N*-diisopropylethylamine (5 mmol) were dissolved in N,N-dimethylformamide (20 ml). After stirring for 5 minutes at room temperature, the amine (2 mmol) was added and the mixture stirred at room temperature overnight. Dichloromethane (50 ml) was added and the solution was washed with 1M sodium hydrogen carbonate solution and then water. Drying and evaporation of the solvent yielded the crude title product which was purified by column chromatography (methanol : dichloromethane, 5:95).

Following the above protocol for the amidation of 2-arylbenzothiazole-6-carboxylic acid (and the general procedure for the synthesis of the acids), the compounds of Examples 4 to 20, 46 and 47 were synthesized:

### Example 4 2-(4-Chloro-2-hydroxyphenyl)-N-[2-(dimethylamino)ethyl]-N-methyl-1,3-benzothiazole-6-carboxamide

From 2-(4-chloro-2-hydroxyphenyl)benzothiazole-6-carboxylic acid (0.062 g, 0.203 mmol) and *N,N,N'*-trimethylethylenediamine (0.051 ml, 0.392 mmol), the title compound was isolated (0.030 g, 38%).
APCI-MS m/z: 390 [MH⁺].

### Example 5 5-Chloro-2-[6-(pyrrolidin-1-ylcarbonyl)-1,3-benzothiazol-2-yl]phenol

From 2-(4-chloro-2-hydroxyphenyl)benzothiazole-6-carboxylic acid (0.060 g, 0.196 mmol) and pyrrolidine (0.033 ml, 0.395 mmol), the title compound was isolated (0.067 g, 95%).
¹H-NMR (400 MHz, CD₂Cl₂): δ 8.13 (1H, d); 8.04 (1H, d); 7.71-7.68 (2H, m); 7.15 (1H, d); 7.01 (1H, dd); 3.73-3.42 (4H, m); 2.04-1.87 (4H, m).
APCI-MS m/z: 359 [MH⁺].

### Example 6 2-(4-Chloro-2-hydroxyphenyl)-N-(3-morpholin-4-ylpropyl)-1,3-benzothiazole-6-carboxamide

From 2-(4-chloro-2-hydroxyphenyl)benzothiazole-6-carboxylic acid (0.060 g, 0.196 mmol) and 3-morpholin-4ylpropan-1-amine (0.058 ml, 0.394 mmol), the title compound was isolated (0.025 g, 29%).
APCI-MS m/z: 432 [MH⁺].

### Example 7 2-(4-Chloro-2-hydroxyphenyl)-N-(2-pyrrolidin-1-ylethyl)-1,3-benzothiazole-6-carboxamide

From 2-(4-chloro-2-hydroxyphenyl)benzothiazole-6-carboxylic acid (0.059 g, 0.193 mmol) and 2-pyrrolidin-1-ylethanamine (0.050 ml, 0.395 mmol), the title compound was isolated (0.018 g, 23 %).
APCI-MS m/z: 402.1 [MH⁺].

### Example 8 5-Chloro-2-(6-{[4-(2-methoxyethyl)piperazin-1-yl]carbonyl}-1,3-benzothiazol-2-yl)phenol

From 2-(4-chloro-2-hydroxyphenyl)benzothiazole-6-carboxylic acid (0.061 g, 0.200 mmol) and 1-(2-methoxyethyl)piperazine (0.062 g, 0.430 mmol), the title compound was isolated (0.061 g, 70%).
APCI-MS m/z: 432.1 [MH⁺].

### Example 9 2-(2-Hydroxy-5-methoxyphenyl)-N-(3-morpholin-4-ylpropyl)-1,3-benzothiazole-6-carboxamide

From 2-(2-hydroxy-5-methoxyphenyl)benzothiazole-6-carboxylic acid (0.059 g, 0.196 mmol) and 3-morpholin-4ylpropan-1-amine (0.059 ml, 0.400 mmol), the title compound was isolated (0.047 g, 56%).
¹H-NMR (400 MHz, acetone-d₆): δ 11.61 (1H, s); 8.66 (1H, s); 8.40 (1H, brs); 8.18-8.08 (2H, m); 7.35 (1H, d); 7.13 (1H, dd); 7.05 (1H, d); 3.87 (3H, s); 3.86-3.72 (2H, m); 3.67-3.55 (8H, m); 3.15-2.62 (2H, m); 2.04-1.95 (2H, m).
APCI-MS m/z: 428 [MH⁺].

### Example 10 4-Methoxy-2-[6-(pyrrolidin-1-ylcarbonyl)-1,3-benzothiazol-2-yl]phenol

From 2-(2-hydroxy-5-methoxyphenyl)benzothiazole-6-carboxylic acid (0.060 g, 0.199 mmol) and pyrrolidine (0.033 ml, 0.395 mmol), the title compound was isolated (0.054 g, 77%).
¹H-NMR (400 MHz, CD₂Cl₂): δ 8.13 (1H, d); 8.04 (1H, d); 7.69 (1H, dd); 7.23 (1H, dt); 7.06 (2H, brs); 3.87 (3H, s); 3.69-3.43 (4H, m); 2.04-1.87 (4H, m).
APCI-MS m/z: 355 [MH⁺].

### Example 11 2-(5-Bromo-2-hydroxyphenyl)-N-[2-(dimethylamino)ethyl]-N-methyl-1,3-benzothiazole-6-carboxamide

From 2-(5-bromo-2-hydroxyphenyl)benzothiazole-6-carboxylic acid (0.396 g, 1.13 mmol) and *N,N,N'*-trimethylethylene diamine (0.300 ml, 2.31 mmol), the title compound was isolated (0.324 g, 66%).
APCI-MS m/z: 433.9 [MH⁺].

### Example 12 4-Fluoro-2-[6-(pyrrolidin-1-ylcarbonyl)-1,3-benzothiazol-2-yl]phenol

From 2-(5-fluoro-2-hydroxyphenyl)benzothiazole-6-carboxylic acid (0.098 g, 0.339 mmol) and pyrrolidine (0.058 ml, 0.691 mmol), the title compound was isolated (0.097 g, 83%).
¹H-NMR (400 MHz, DMSO-d₆): δ 11.43 (1H, s); 8.34 (1H, d); 8.08 (1H, d); 8.03 (1H, dd); 7.67 (1H, dd); 7.30 (1H, dt); 7.10 (1H, dd); 3.53-3.43 (4H, m); 1.91-1.81 (4H, m). APCI-MS m/z: 343.1 [MH⁺].

### Example 13 5-Methoxy-2-[6-(pyrrolidin-1-ylcarbonyl)-1,3-benzothiazol-2-yl]phenol

From 2-(2-hydroxy-4-methoxyphenyl)benzothiazole-6-carboxylic acid (0.062 g, 0.206 mmol) and pyrrolidine (0.033 ml, 0.395 mmol), the title compound was isolated (0.059 g, 81%).
¹H-NMR (400 MHz, CD₂Cl₂): δ 8.08 (1H, d); 7.97 (1H, d); 7.67-7.64 (2H, m); 6.62-6.57 (2H, m); 3.89 (3H, s); 3.70-3.45 (4H, m); 2.02-1.90 (4H, m).
APCI-MS m/z: 355 [MH⁺].

### Example 14 Methyl N-{[2-(2-hydroxyphenyl)-1,3-benzothiazol-6-yl]carbonyl}serinate

From 2-(2-hydroxyphenyl)benzothiazole-6-carboxylic acid (0.200 g, 0.737 mmol) and serine methyl ester hydrochloride (0.229 g, 1.47 mmol), the title compound was isolated (0.116 g, 42%).
¹H-NMR (400 MHz, DMSO-d₆): δ 8.71 (1H, d); 8.67 (1H, d); 8.26 (1H, dd); 8.12 (1H, d); 8.04 (1H, dd); 7.44 (1H, dt); 7.10 (1H, d); 7.03 (1H, t); 5.08 (1H, t); 4.62-4.56 (1H, m); 3.83 (2H, t); 3.67 (3H, s).
APCI-MS m/z: 373.1 [MH⁺].

### Example 15 2-[6-(Pyrrolidin-1-ylcarbonyl)-1,3-benzothiazol-2-yl]phenol

From 2-(2-hydroxyphenyl)benzothiazole-6-carboxylic acid (0.062 g, 0.229 mmol) and pyrrolidine (0.037 ml, 0.443 mmol), the title compound was isolated (0.064 g, 86%).
¹H-NMR (400 MHz, CD₂Cl₂): δ 8.12 (1H, d); 8.04 (1H, d); 7.77 (1H, dd); 7.69 (1H, dd); 7.45 (1H, dt); 7.12 (1H, d); 7.02 (1H, dt); 3.70-3.45 (4H, m); 2.05-1.90 (4H, m).
APCI-MS m/z: 325 [MH⁺].

### Example 16 2-(5-Chloro-2-hydroxyphenyl)-N-(3-morpholin-4-ylpropyl)-1,3-benzothiazole-6-carboxamide

From 2-(5-chloro-2-hydroxyphenyl)benzothiazole-6-carboxylic acid (0.061 g, 0.200 mmol) and 3-morpholin-4ylpropan-1-amine (0.058 ml, 0.394 mmol), the title compound was isolated (0.059 g, 68%).
¹H-NMR (400 MHz, DMSO-d₆): δ 11.73 (1H, s); 9.62 (1H, brs); 8.62 (1H, d); 8.29 (1H, d); 8.14 (1H, d); 8.01 (1H, dd); 7.48 (1H, dd); 7.14 (1H, d); 3.99 (2H, d); 3.65 (2H, t); 3.24-3.00 (6H, m); 2.01-1.90 (2H, m); 1.26 (2H, t).
APCI-MS m/z: 432 [MH⁺].

### Example 17 3-Methoxy-2-[6-(pyrrolidin-1-ylcarbonyl)-1,3-benzothiazol-2-yl]phenol

From 2-(2-hydroxy-6-methoxyphenyl)benzothiazole-6-carboxylic acid (0.060 g, 0.199 mmol) and pyrrolidine (0.033 ml, 0.395 mmol), the title compound was isolated (0.065 g, 92%).
¹H-NMR (400 MHz, CD₂Cl₂): δ 8.15 (1H, d); 8.04 (1H, d); 7.69 (1H, dd); 7.40 (1H, t); 6.79 (1H, d); 6.61 (1H, d); 4.11 (3H, s); 3.62-3.55 (4H, m); 2.00-1.94 (4H, m).
APCI-MS m/z: 355 [MH⁺].

### Example 18 N,N-Diethyl-2-(2-hydroxy-4-methoxyphenyl)-1,3-benzothiazole-6-carboxamide

From 2-(2-hydroxy-4-methoxyphenyl)benzothiazole-6-carboxylic acid (0.061 g, 0.202 mmol) and diethylamine (0.050 ml, 0.483 mmol), the title compound was isolated (0.007 g, 10%).
APCI-MS m/z: 357.1 [MH⁺].

### Example 19 N-[2-Hydroxy-1-(hydroxymethyl)ethyl]-2-(2-hydroxy-4-methoxyphenyl)-1,3-benzothiazole-6-carboxamide

From 2-(2-hydroxy-4-methoxyphenyl)benzothiazole-6-carboxylic acid (0.200 g, 0.664 mmol) and serinol (0.130 g, 1.43 mmol), the title compound was isolated (0.017 g, 7%).
APCI-MS m/z: 375.1 [MH⁺]^{.}.

### Example 20 2-[6-(Pyrrolidin-1-ylcarbonyl)-1,3-benzothiazol-2-yl]pyridin-3-ol

From 2-(3-hydroxypyridine-2-yl)benzothiazole-6-carboxylic acid (0.074 g, 0.272 mmol) and pyrrolidine (0.045 ml, 0.544 mmol), the title compound was isolated (0.055 g, 62%). ¹H-NMR (400 MHz, CD₂Cl₂): δ 8.29 (1H, dd); 8.17 (1H, d); 8.09 (1H, d); 7.71 (1H, dd); 7.50 (1H, d); 7.41 (1H, dd); 3.72-3.44 (4H, m); 2.05-1.90 (4H, m).
APCI-MS m/z: 326 [MH⁺].

### Example 21 Methyl 4-{[2-(6-{[[2-6-(dimethylamino)ethyl](methyl)amino]-carbonyl}-1,3-benzothiazol-2-yl)-3-hydroxyphenoxy]methyl}benzoate

In dry N,N-dimethylformamide (15 ml), 2,6-dihydroxybenzaldehyde (0.293 g, 2.12 mmol) and Cs₂CO₃ (0.828 g, 2.54 mmol) were dissolved. Following the addition of methyl 4-(bromomethyl)benzoate (0.490 g, 2.14 mmol) the mixture was stirred at room temperature overnight. After filtration, the solvent was evaporated, the residue dissolved in dichloromethane and washed with saturated aqueous sodium hydrogen carbonate solution. Drying (Na₂SO₄) and evaporation afforded crude material that was purified by flash chromatography (ethyl acetate : heptane, 1:3), giving methyl 4-[(2-formyl-3-hydroxyphenoxy)methyl]benzoate as a white solid (0.385 g, 63%).
¹H-NMR (400 MHz, DMSO-d₆): δ 10.4 (1H, s); 7.99 (2H, d); 7.65 (2H, d); 7.52 (1H, t); 6.68 (1H, d); 6.55 (1H, d); 5.35 (2H, s); 3.85 (3H, s).
This aldehyde was then reacted with 4-amino-3-mercaptobenzoic acid according to General Method 1 to give the corresponding benzothiazole derivative. Further reaction with *N,N,N*'-trimethylethylenediamine using the general amidation procedure then gave the title compound.
APCI-MS m/z: 520.1 [MH⁺].

### Example 22 5-Ethoxy-4-[6-(pyrrolidin-1-ylcarbon)-1,3-benzothiazol-2-yl]pyridin-3-ol

A mixture of 3,5-diethoxy-4-[6-(pyrrolidin-1-ylcarbonyl)-1,3-benzothiazol-2-yl]pyridine (prepared according to the general method 1) (0.200 g, 0.503 mmol) and BBr₃ (1 ml, 1M solution in dichloromethane) was refluxed overnight in dichloromethane (20 ml). After cooling to room temperature, water (5 ml) was added. The organic phase was washed with brine and dried (Na₂SO₄). The crude material isolated upon evaporation was purified by column chromatography (methanol : dichloromethane, 5:95), delivering the title product (0.046 g, 25%).
¹H-NMR (400 MHz, CD₂Cl₂): δ 13.48 (1H, s); 8.23 (1H, s); 8.20 (1H, s); 8.13 (1H, d); 8.05 (1H, s); 7.74 (1H, dd); 4.46 (2H, q); 3.67 (2H, t); 3.50 (2H, t); 2.03-1.90 (4H, m); 1.72 (3H, t).
APCI-MS m/z: 370 [MH⁺].

### Example 23 N-[2-(Dimethylamino)ethyl]-2-(4-hydroxy-1,1'-biphenyl-3-yl)-N-methyl-1,3-benzothiazole-6-carboxamide

A mixture of 2-(5-bromo-2-hydroxyphenyl)-N-[2-(dimethylamino)ethyl]-N-methyl-1,3-benzothiazole-6-carboxamide (0.048 g, 0.11 mmol), phenyl boronic acid (0.039 g, 0.32 mmol), [1,1'-bis(diphenylphosphino)ferrocene]dichloropalladium(II) dichloromethane complex (0.012 g, 0.0147 mmol) and 2M sodium carbonate solution (0.29 ml, 0.58 mmol) was heated at 100 °C in dioxane (4 ml) and ethanol (0.5 ml) overnight. After cooling to room temperature and filtration, the solution was washed with 1M sodium hydrogen carbonate solution, dried (Na₂SO₄) and evaporated to give the crude product. Purification by column chromatography (methanol : dichloromethane, 5:95) delivered the title product (0.015 g, 32%).
APCI-MS m/z: 432.1 [MH⁺].

### Example 24 N-[2-(Dimethylamino)ethyl]-2-(4-hydroxy-3'-methoxy-1,1'-biphenyl-3-yl)-N-methyl-1,3-benzothiazole-6-carboxamide

A mixture of 2-(5-bromo-2-hydroxyphenyl)-N-[2-(dimethylamino)ethyl]-N-methyl-1,3-benzothiazole-6-carboxamide (0.050 g, 0.115 mmol), 3-methoxyphenyl boronic acid (0.048 g, 0.345 mmol), [1,1'-bis(diphenylphosphino)ferrocene] dichloropalladium(II) dichloromethane complex (0.010 g, 0.0122 mmol)) and 2M sodium carbonate solution (0.29 ml, 0.58 mmol) was heated at 100 °C in dioxane (4 ml) and ethanol (0.5 ml) overnight. After cooling to room temperature and filtration, the solution was washed with 1M sodium hydrogen carbonate solution, dried (Na₂SO₄) and evaporated to give the crude product. Purification by column chromatography (methanol : dichloromethane, 5:95) delivered the title product (0.017 g, 32%).
APCI-MS m/z: 462.1 [MH⁺].

### General procedure for the reduction of 2-arylbenzothiazolecarboxamides to the corresponding amines or alcohols

The 2-arylbenzothiazole-6-carboxamide (0.2 mmol) was dissolved in tetrahydrofuran. After cooling to 0 °C, a 1M solution of lithium aluminium hydride in tetrahydrofuran (0.4 mmol) was added dropwise. The ice-bath was removed and the reaction mixture stirred at room temperature until completion of the reduction (LC-MS). Water (0.5 ml) was added followed by dichloromethane (30 ml). After washing with brine, drying and evaporation, the crude title product was purified by column chromatography (methanol : dichloromethane, 5:95).

Following this general procedure (and the general procedures for the synthesis of the corresponding amides), the amines of Examples 25 to 44 were synthesised:

### Example 25 5-Chloro-2-(6-{[[2-(dimethylamino)ethyl](methyl)amino]methyl}-1.3-benzothiazol-2-yl)phenol

From 2-(4-chloro-2-hydroxyphenyl)-N-[2-(dimethylamino)ethyl]-N-methyl-1,3-benzothiazole-6-carboxamide (0.026 g, 0.067 mmol), the title compound was isolated (0.007 g, 28%).
¹H-NMR (400 MHz, CD₂Cl₂): δ 7.97 (1H, s); 7.96 (1H, d); 7.67 (1H, d); 7.53 (1H, dd); 7.12 (1H, d); 6.98 (1H, dd); 3.69 (2H, s); 2.64-2.55 (4H, m); 2.32 (6H, s); 2.27 (3H, s). APCI-MS m/z: 376.2 [MH⁺].

### Example 26 5-Chloro-2-(6-{[4-(2-methoxyethyl)piperazin-1-yl]methyl}-1,3-benzothiazol-2-yl)phenol

From 5-chloro-2-(6-{[4-(2-methoxyethyl)piperazin-1-yl]carbonyl}-1,3-benzothiazol-2-yl)phenol (0.060 g, 0.139 mmol), the title compound was isolated (0.022 g, 38%).
APCI-MS m/z: 418.3 [MH⁺].

### Example 27 5-Chloro-2-[6-(pyrrolidin-1-ylmethyl)-1,3-benzothiazol-2-yl]phenol

From 5-chloro-2-[6-(pyrrolidin-1-ylcarbonyl)-1,3-benzothiazol-2-yl]phenol (0.065 g, 0.181 mmol), the title compound was isolated (0.028 g, 45%).
¹H-NMR (400 MHz, CD₂Cl₂): δ 12.72 (1H, brs); 8.03 (1H, s); 7.97 (1H, d); 7.68 (1H, d); 7.57 (1H, d); 7.13 (1H, d); 6.99 (1H, dd); 3.87 (2H, s); 2.74-2.60 (4H, m); 1.91-1.82 (4H, m).
APCI-MS m/z: 345.1 [MH⁺].

### Example 28 4-Fluoro-2-[6-(pyrrolidin-1-ylmethyl)-1,3-bentothiazol-2-yl]phenol

From 4-fluoro-2-[6-(pyrrolidin-1-ylcarbonyl)-1,3-benzothiazol-2-yl]phenol (0.090 g, 0.263 mmol), the title compound was isolated (0.044 g, 51 %).
¹H-NMR (400 MHz, DMSO-d₆): δ 8.05 (1H, s); 8.01-7.95 (2H, m); 7.49 (1H, dd); 7.27 (1H, ddd); 7.09 (1H, dd); 3.74 (2H, s); 2.53-2.44 (4H, m); 1.76-1.67 (4H, m).
APCI-MS m/z: 329.2 [MH⁺].

### Example 29 2-[6-(Pyrrolidin-1-ylmethyl)-1,3-benzothiazol-2-yl]phenol

From 2-[6-(pyrrolidin-1-ylcarbonyl)-1,3-benzothiazol-2-yl]phenol (0.062 g, 0.191 mmol), the title compound was isolated (0.028 g, 45%).
¹H-NMR (400 MHz, CD₂Cl₂): δ 12.43 (1H, brs); 8.01-7.95 (2H, m); 7.75 (1H, d); 7.55 (1H, d); 7.42 (1H, t); 7.10 (1H, d); 7.00 (1H, t); 3.86 (2H, s); 2.72-2.60 (4H, m); 1.90-1.82 (4H, m).
APCI-MS m/z: 311 [MH⁺].

### Example 30 3-Methoxy-2-[6-(pyrrolidin-1-ylmethyl)-1,3-benzothiazol-2-yl]phenol

From 3-methoxy-2-[6-(pyrrolidin-1-ylcarbonyl)-1,3-benzothiazol-2-yl]phenol (0.063 g, 0.178 mmol), the title compound was isolated (0.035 g, 58%).
¹H-NMR (400 MHz, CD₂Cl₂): δ 7.99 (1H, s); 7.96 (1H, d); 7.54 (1H, dd); 7.35 (1H, t); 6.75 (1H, dd); 6.59 (1H, dd); 4.08 (3H, s); 3.89 (2H, s); 2.75-2.67 (4H, m); 1.91-1.84 (4H, m).
APCI-MS m/z: 341.1 [MH⁺].

### Example 31 4-Methoxy-2-[6-(pyrrolidin-1-ylmethyl)-1,3-benzothiazol-2-yl]phenol

From 4-methoxy-2-[6-(pyrrolidin-1-ylcarbonyl)-1,3-benzothiazol-2-yl]phenol (0.052 g, 0.147 mmol), the title compound was isolated (0.033 g, 66%).
¹H-NMR (400 MHz, CD₂Cl₂): δ 12.00 (1H, brs); 8.01 (1H, s); 7.97 (1H, d); 7.55 (1H, dd); 7.22 (1H, t); 7.03 (2H, d); 3.87 (2H, s); 2.72-2.64 (4H, m); 1.90-1.84 (4H, m).
APCI-MS m/z: 341.1 [MH⁺].

### Example 32 4-Bromo-2-[6-(pyrrolidin-1-ylmethyl)-1,3-benzothiazol-2-yl]phenol

From 4-bromo-2-[6-(pyrrolidin-1-ylcarbonyl)-1,3-benzothiazol-2-yl]phenol (0.062 g, 0.154 mmol), the title compound was isolated (0.024 g, 40%).
¹H-NMR (400 MHz, CD₂Cl₂): δ 7.99 (1H, s); 7.97 (1H, d); 7.85 (1H, d); 7.56 (1H, dd); 7.48 (1H, dd); 7.01 (1H, d); 3.83 (2H, s); 2.67-2.58 (4H, m); 1.88-1.81 (4H, m).
APCI-MS m/z: 378.9 [MH⁺].

### Example 33 5-Methoxy-2-[6-(pyrrolidin-1-ylmethyl)-1,3-benzothiazol-2-yl]phenol

From 5-methoxy-2-[6-(pyrrolidin-1-ylcarbonyl)-1,3-benzothiazol-2-yl]phenol (0.057 g, 0.161 mmol), the title compound was isolated (0.014 g, 26%).
¹H-NMR (400 MHz, CD₂Cl₂): δ 7.96 (1H, s); 7.92 (1H, d); 7.62 (1H, d); 7.51 (1H, dd); 6.61-6.54 (2H, m); 3.98 (2H, s); 3.88 (3H, s); 2.90-2.81 (4H, m); 1.97-1.90 (4H, m).
APCI-MS m/z: 341.2 [MH⁺].

### Example 34 4-Chloro-2-[6-(pyrrolidin-1-ylmethyl)-1,3-benzothiazol-2-yl]phenol

From 4-chloro-2-[6-(pyrrolidin-1-ylcarbonyl)-1,3-benzothiazol-2-yl]phenol (0.063 g, 0.176 mmol), the title compound was isolated (0.035 g, 58%).
¹H-NMR (400 MHz, CD₂Cl₂): δ 12.48 (1H, brs); 8.10 (1H, s); 8.00 (1H, d); 7.73 (1H, d); 7.64 (1H, d); 7.37 (1H, dd); 7.07 (1H, d); 3.95 (2H, s); 2.90-2.64 (4H, m); 1.99-1.85 (4H, m).
APCI-MS m/z: 345 [MH⁺].

### Example 35 2-{6-[(Diethylamino)methyl]-1,3-benzothiazol-2-yl}-5-methoxyphenol

From N,N-diethyl-2-(2-hydroxy-4-methoxyphenyl)-1,3-benzothiazole-6-carboxamide (0.019 g, 0.053 mmol), the title compound was isolated (0.009 g, 49%).
¹H-NMR (400 MHz, CDCl₃): δ 7.87 (1H, s); 7.85 (1H, d); 7.57 (1H, d); 7.45 (1H, d); 6.60 (1H, d); 6.53 (1H, dd); 3.86 (2H, s); 2.57 (4H, q); 1.08 (6H, t).
APCI-MS m/z: 343.1 [MH⁺].

### Example 36 5-Ethoxy-4-[6-(pyrrolidin-1-ylmethyl)-1,3-benzothiazol-2-yl]pyridin-3-ol

From 5-ethoxy-4-[6-(pyrrolidin-1-ylcarbonyl)-1,3-benzothiazol-2-yl]pyridin-3-ol (0.038 g, 0.103 mmol), the title compound was isolated (0.011 g, 30%).
¹H-NMR (400 MHz, CD₂Cl₂): δ 13.59 (1H, s); 8.28-7.98 (4H, m); 7.58 (1H, d); 4.43 (2H, q), 3.81 (2H, s); 2.64-2.52 (4H, m); 1.85-1.79 (4H, m); 1.70 (3H, t).
APCI-MS m/z: 356 [MH⁺].

### Example 37 4-Chloro-2-(6-{[(3-morpholin-4-ylpropyl)amino]methyl}-1,3-benzothiazol-2-yl)phenol

From 2-(5-chloro-2-hydroxyphenyl)-N-(3-morpholin-4-ylpropyl)-1,3-benzothiazole-6-carboxamide (0.056 g, 0.130 mmol), the title compound was isolated (0.005 g, 9%).
APCI-MS m/z: 418 [MH⁺].

### Example 38 2-[6-(Pyrrolidin-1-ylmethyl)-1,3-benzothiazol-2-yl]pyridin-3-ol

From 2-[6-(pyrrolidin-1-ylcarbonyl)-1,3-benzothiazol-2-yl]pyridin-3-ol(0.070 g, 0.215 mmol), the title compound was isolated (0.038 g, 57%).
¹H-NMR (400 MHz, CD₂Cl₂): δ 12.08 (1H, s); 8.26 (1H, dd); 8.02-7.98 (2H, m); 7.55 (1H, dd); 7.47-7.34 (2H, m); 3.81 (2H, s); 2.65-2.53 (4H, m); 1.88-1.79 (4H, m).
APCI-MS m/z: 312 [MH⁺].

### Example 39 2-[5-(Pyrrolidin-1-ylmethyl)-1,3-benzothiazol-2-yl]phenol

From 2-[5-(pyrrolidin-1-ylcarbonyl)-1,3-benzothiazol-2-yl]phenol (0.010 g, 0.031 mmol), the title compound was isolated (0.005 g, 52%).
APCI-MS m/z: 311 [MH⁺].

### Example 40 5-Chloro-2-[6-(piperidin-1-ylmethyl)-1,3-benzothiazol-2-yl]phenol

APCI-MS m/z: 359 [MH⁺].

### Example 41 5-Chloro-2-[6-(4-methylpiperazin-1-ylmethyl)-1,3-benzothiazol-2-yl]phenol

APCI-MS m/z: 374.1 [MH⁺].

### Example 42 5-Chloro-2-{6-[(Diethylamino)methyl]-1,3-benzothiazol-2-yl}phenol

APCI-MS m/z: 347 [MH⁺].

### Example 43 5-Chloro-2-{6-[(dimethylamino)methyl]-1,3-benzothiazol-2-yl}phenol

APCI-MS m/z: 319 [MH⁺].

### Example 44 2-[6-(Hydroxymethyl)-1,3-benzothiazol-2-yl]phenol

From 2-(2-hydroxyphenyl)-N-phenyl-benzothiazole-6-carboxamide (0.042 g, 0.121 mmol), the title compound was isolated (0.003 g, 10%).
APCI-MS m/z: 258.1 [MH⁺].

### Example 45 2-(6-Amino-1,3-benzothiazol-2-yl)-4-methoxyphenol

A mixture of 2-(2-hydroxy-5-methoxyphenyl)-1,3-benzothiazole-6-carboxylic acid (0.502 g, 1.67 mmol) and triethylamine (0.35 ml, 2.51 mmol) was dissolved in N,N-dimethylformamide (10 ml). Diphenylphosphorylazide (0.54 ml, 2.51 mmol) was added via syringe. The mixture was stirred at room temperature for 3h, after which water (2 ml) was added and the solution heated at 100 °C for 1h. After cooling the reaction mixture was diluted with dichloromethane (30 ml), and washed with water and 1M sodium hydrogen carbonate solution. The dichloromethane solution was extracted with 1M sodium hydroxide solution, the pH adjusted to 9 and the basic water phase was re-extracted with dichloromethane. After drying (Na₂SO₄) and filtration the title compound was isolated (0.004g, 0.9%).
¹H-NMR (400 MHz, DMSO-d₆): δ 11.11 (1H, s); 7.71 (1H, d); 7.42 (1H, s); 7.10 (1H, d); 6.96 (2H, d); 6.81 (1H, dd); 5.49 (2H, s); 3.78 (3H, s).
APCI-MS m/z: 373.1 [MH⁺].

### Example 46 N,N Dimethyl-2-(2-hydroxy-4-methoxyphenyl)-1,3-benzothiazole-6-carboxamide

APCI-MS m/z: 329.1 [MH⁺].

### Example 47 N,N-Dimethyl-2-(2-hydroxyphenyl)-1,3-benzothiazole-6-carboxamide

APCI-MS m/z: 299.1 [MH⁺].

### Example 48 3-[6-(Pyrrolidin-1-ylcarbonyl)-1,3-benzothiazol-2-yl]pyrazin-2-amine

### 3-Amino-pyrazine-2-carbaldehyde:

3-Aminopyrazine-2-carboxylic acid methyl ester (3.0 g, 20 mmol) was suspended in tetrahydrofuran (100 ml) under a nitrogen atmosphere and cooled to 0 °C. Lithium aluminium hydride as a 1.0M solution in tetrahydrofuran (20 ml, 20 mmol) was added dropwise over 30 minutes whereupon the solution was stirred for another 30 minutes. Saturated aqueous ammonium chloride solution (100 ml) was carefully added (evolution of gas) and the resulting mixture acidified (pH 1) with 4M hydrochloric acid, shaken (5 minutes) and then basified again (pH 10) with saturated aqueous sodium hydrogen carbonate solution. The mixture was extracted with diethyl ether (3 x 150 ml) and the combined organic phase dried (MgSO₄) and concentrated *in vacuo.* The yellow oily residue was applied to column chromatography (silica gel/dichloromethane/methanol) and the first fraction was concentrated *in vacuo* to yield the aldehyde as pale yellow crystals (689 mg, 28 %).
¹H-NMR (400 MHz, DMSO-d₆): δ 9.90 (1H, s); 8.31 (1H, d); 8.02 (1H, d); 7.67 (2H, bs). APCI-MS m/z: 124 (MH⁺).
Following the protocol for the preparation of 2-arylbenzothiazole-6-carboxylic acids (Method 1), 3-amino-pyrazine-2-carbaldehyde and 4-amino-3-mercaptobenzoic acid were reacted together to give 2-(3-aminopyrazin-2-yl)-benzothiazole-6-carboxylic acid in 19% yield. This product (136 mg, 0.5 mmol) and pyrrolidine (360 mg, 5 mmol) were mixed in N,N-dimethylformamide (3 ml). HBTU (209 mg, 0.55 mmol) was added and the resulting mixture heated (50 °C) for 1h. The reaction mixture was diluted with ethyl acetate (20 ml) and washed with saturated aqueous sodium hydrogen carbonate solution (2 x 20 ml) and brine (10 ml). The organic phase was concentrated *in vacuo* and the residue chromatographed on silica using ethyl acetate as eluent. This afforded the amide as an off-white powder (33 mg, 20%).
¹H-NMR (400 MHz, CDCl₃): δ 8.15 (1H, s); 8.10-8.08 (2H, m); 7.98 (1H, s); 7.70 (1H, d); 7.62 (0.5H, s); 7.59 (0.25H, s); 7.47 (0.25 H, s); 4.4 (1H, bs); 3.7-3.4 (4H, m); 2.0-1.9 (4H, m).

### Example 49 2-(4-Chloro-2-hydroxyphenyl)-1,3-benzothiazol-6-ol

2-(4-Chloro-2-methoxyphenyl)-6-methoxybenzothiazole (2.48 g, 8.1 mmol) [prepared starting from 4-methoxyaniline and 4-chloro-2-hydroxybenzoic acid by the general Method 2 described above], was dissolved in dichloromethane (200 ml) and BBr₃ (1M in dichloromethane, 49 ml) was added. The reaction mixture was stirred overnight at room temperature. The reaction mixture was diluted with dichloromethane (200 ml) and washed with saturated aqueous sodium hydrogen carbonate solution (300 ml) and brine (300 ml). The organic layer was dried (Na₂SO₄) and evaporated to dryness. Recrystallization from dichloromethane/methanol yielded the title compound (1.955 g, 87%).
APCI-MS m/z: 278.0, 280.0 [MH⁺].

### Example 50 5-Chloro-2-[6-(2-hydroxy-3-pyrrolidin-1- yl-propoxy)-1,3-benzothiazol-2-yl]-phenol

### a) 5-Chloro-2-[6-(2,3-epoxypropoxy)-1,3-benzothiazol-2-yl]phenol

2-(4-Chloro-2-hydroxyphenyl)-6-hydroxy-1,3-benzothiazole (60 mg, 0.22 mmol) was dissolved in dry tetrahydrofuran (10 ml) and sodium hydride (60% in mineral oil, 24 mg, 1.67 mmol) was added followed by epibromohydrin (22 µl, 0.26 mmol). The reaction mixture was stirred for 24h at 55 °C before it was quenched with water (200 µl), diluted with ethyl acetate (50 ml) and washed with 1.5 % hydrochloric acid (50 ml) followed by saturated aqueous sodium hydrogen carbonate solution (50 ml). Purification by HPLC-C₁₈ yielded the title epoxide (6 mg, 8%).
¹H-NMR (400 MHz, DMSO-d₆): δ 11.87 (1H, bs); 8.16 (1H, d); 7.95 (1H, d); 7.72 (1H, d) ; 7.17 (1H, dd); 7.10 (1H, d); 7.05 (1H, dd); 4.43 (1H, dd); 3.95 (1H,dd); 3.39 (1H, m); 2.87 (1H, t); 2.75 (1H, dd).
APCI-MS m/z: 333.9, 335.9 [MH⁺].

### 5-Chloro-2-[6-(2-hydroxy-3-pyrrolidin-1-yl-propoxy)-1,3-benzothiazol-2-yl]-phenol

5-Chloro-2-[6-(2,3-epoxypropoxy)-1,3-benzothiazol-2-yl]phenol (6 mg, 0,018 mmol) was dissolved in dioxane (2 ml) and diluted with ethanol (30 ml). Pyrrolidine (100 µl, 1.2 mmol) was added and the mixture was stirred at 70 °C for 2h. After evaporation of the solvent and excess reagent the product was purified by HPLC-C₁₈. Yield 7 mg (95%).
APCI-MS m/z: 405.1, 407.1 [MH⁺].

### Example 51 5-Chloro-2-[5-(pyrrolidin-1-ylcarbonyl)-1,3-benzothiazol-2-yl]phenol

### a) 2-(4-Chloro-2-hydroxyphenyl)-1,3-benzothiazole-5-carboxylic acid

This compound was prepared by the general method described above. The regioisomers were separated by BPLC-C₁₈.
¹H-NMR (400 MHz, DMSO-d₆): δ 13.14 (1H, bs); 11.93 (1H, bs); 8.54 (1H, s); 8.31 (1H, d); 8.28 (1H, d); 7.98 (1H, dd); 7.11 (2H, m).
APCI-MS m/z: 306.1 [MH⁺].

### b) 5-Chloro-2-[5-(pyrrolidin-1-ylcarbonyl)-1,3-benzothiazol-2-yl]phenol

2-(4-Chloro-2-hydroxyphenyl)-1,3-benzothiazole-5-carboxylic acid (25 mg, 0.082 mmol) was dissolved in NMP (1.5 ml) and mixed with HBTU (34 mg, 0,090 mmol), DIEA (28 µl, 0.16 mmol) and pyrrolidine (10 µl, 0.16 mmol). The mixture was stirred at room temperature overnight. Purification was made by HPLC-C₁₈ and yielded the title compound (7mg, 23%).
APCI-MS m/z: 359.2, 361.2 [MH⁺].

### Example 52 2-[5-(Pyrrolidin-1-ylcarbonyl)-1,3-benzothiazol-2-yl]phenol

The title compound was prepared by a route analogous to that used for Example 51.
APCI-MS m/z: 325.2 [MH⁺].

### Example 53 N,N-Dimethyl-2-(2-hydroxyphenyl)-1,3-benzothiazole-5-carboxamide

The title compound was prepared by a route analogous to that used for Example 51.
APCI-MS m/z: 299.2 [MH⁺].

### Example 54 5-Chloro-2-[5-(pyrrolidin-1-ylmethyl)-1,3-benzothiazol-2-yl]phenol

5-Chloro-2-[5-(pyrrolidin-1-ylcarbonyl)-1,3-benzothiazol-2-yl]phenol (5 mg, 0.014 mmol) was dissolved in dry tetrahydrofuran (2 ml) under an inert atmosphere at 0 °C. Lithium aluminium hydride (28 µl, 1M in tetrahydrofuran) was added and the mixture was stirred for 1 h before it was quenched with methanol (100 µl). The product was purified by HPLC-C₁₈ giving the title compound (2 mg, 41 %).
APCI-MS m/z: 345.1, 347.1 [MH⁺].

### Example 55 2-{5-[(Dimethylamino)methyl]-1,3-benzothiazol-2-yl}phenol

The title compound was prepared by a route analogous to those used for Examples 51 and 54.
APCI-MS m/z: 285.1 [MH⁺].

### Example 56 2-(2-Hydroxyphenyl)-N-(3-morpholin-4-ylpropyl)-1,3-benzothiazole-7-carboxamide

### a) 2-(2-Hydroxyphenyl)-1,3-benzothiazole-7-carboxylic acid

This compound was prepared according to the general method described above. The regioisomers were separated by HPLC-C₁₈.
¹H-NMR (400 MHz, DMSO-d₆): δ 13.71(1H, bs); 11.56 (1H, bs); 8.30 (1H, d); 8.23 (1H, d); 8.08 (1H, d); 7.67 (1H, t); 7.43 (1H, dt); 7.10 (1H, d); 7.02 (1H, t).
APCI-MS m/z: 272.1 [MH⁺].

### b) 2-(2-Hydroxyphenyl)-N-(3-morpholin-4-ylpropyl)-1,3-benzothiazole-7-carboxamide

The title compound was prepared by the same route as Example 51 (b). APCI-MS m/z: 398.2 [MH⁺].

### Screen

### Itk LANCE TRF assay

The Itk kinase assay utilized recombinant human Itk kinase domain fused with GST (Glutathione S-Transferase). The protein was expressed in High five insect cells, purified in one step on an affinity chromatography glutathione column and stored in 50 mM Tris/HCl (pH 7.6), 150 mM NaCl, 5% (w/v) mannitol, 1 mM DTT, 30% glycerol at -70 °C. The kinase substrate used in the assay was a biotinylated peptide derived from the Src-optimal substrate (Nair *et al,* J. Med. Chem., 38: 4276, 1995; biotin-AEEEIYGEFEAKKKK).
The assay additions were as follows: Test compounds (or controls; 1 µL in 100% DMSO) were added to black 96-well flat-bottomed plates (Greiner 655076) followed by 20 µL Itk in assay buffer and the reaction was started by adding 20 µL ATP and peptide substrate in assay buffer. The assay buffer constitution during phosphorylation was: 50 mM HEPES (pH 6.8), 10 mM MgCl₂, 0.015% Brij 35, 1 mM DTT, 10% glycerol, 160 ng/well Itk, 2 µM peptide substrate and 50 µM ATP. The assay was stopped after 50 minutes (RT) by adding 150 µL ice-cold Stop solution (50 mM Tris/HCl, pH 7.5, 10 mM EDTA, 0.9% NaCl and 0.1% BSA) together with LANCE reagents (2 nM PT66-Eu³⁺, Wallac AD0069 and 5 µg/ml Streptavidin-APC, Wallac AD0059. Both concentrations were final in stopped assay solution). The plates were measured on a Wallac 1420 Victor 2 instrument with TRF settings after 1h incubation, and the ratio (665 signal/615 signal)^{*} 10000 was used to calculate the inhibition values. IC₅₀ values were determined using XLfit.

When tested in the above screens, the compounds of Examples 1 to 56 gave IC₅₀ values for inhibition of Itk activity of less than 25 µM, indicating that the compounds of the invention are expected to possess useful therapeutic properties.
Representative results are shown in the following Table:

| **Compound** | **Inhibition of Kinase Itk (IC**_{**50**} **µM)** |
|---|---|
| Example 6 | 0.94 |
| Example 34 | 1.47 |
| Example 44 | 3.04 |

## Claims

1. The use of a compound of formula (I) 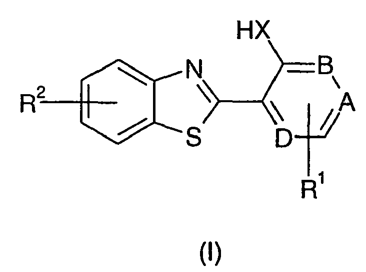 wherein:
A and D independently represent CH or N; and
either B represents CH and X represents O;
or B represents N and X represents NH;
R¹ represents hydrogen, halogen, C1 to 6 alkyl, C1 to 6 alkoxy, aryl or benzyloxy; said aryl or benzyloxy group being optionally further substituted by a group selected from C1 to 6 alkyl, C1 to 6 alkoxy, halogen and carbomethoxy;
R² represents hydroxy, amino, C1 to 6 alkyl, C1 to 6 alkoxy, carbamoyl (-CONR³R⁴) or -COOH; said alkyl or alkoxy group being optionally further substituted by one or more groups independently selected from hydroxy and NR⁵R⁶;
R³ R⁴, R⁵ and R⁶ each independently represent hydrogen or C1 to 6 alkyl; said alkyl group being optionally further substituted by one or more substituents selected independently from hydroxy, C1 to 6 alkoxy, NR⁷R⁸ and C1 to 6 alkoxycarbonyl;
or the group NR³R⁴ or the group NR⁵R⁶ may together represent a 5 to 7 membered azacyclic ring optionally incorporating one further heteroatom selected from O and NR⁹;
R⁷ and R⁸ independently represent hydrogen or C1 to 6 alkyl; or the group NR⁷R ⁸ together represents a 5 to 7 membered azacyclic ring optionally incorporating one further heteroatom selected from O and NR⁹;
R⁹ represents hydrogen or C1 to 6 alkyl; said alkyl group being optionally further substituted by C1 to 6 alkoxy;
or a pharmaceutically acceptable salts thereof, in the manufacture of a medicament for the treatment or prophylaxis of diseases or conditions in which inhibition of kinase Itk activity is beneficial.

2. The use according to Claim 1 of a compound of formula (I) or a pharmaceutically acceptable salt thereof, in the manufacture of a medicament for the treatment or prophylaxis of Th2-driven and/or mast cell-driven and/or basophil driven diseases or conditions.

3. The use according to Claim 2 wherein the disease is asthma.

4. The use according to Claim 2 wherein the disease is allergic rhinitis.

5. The use according to any one of Claims 1 to 4 wherein B in formula (I) represents CH and X represents O.

6. The use according to any one of Claims 1 to 5 wherein R² in formula (I) represents -CONR³R⁴ or R² represents C1 to 6 alkyl substituted by NR⁵R⁶.

7. The use according to any one of Claims 1 to 4 wherein the compound of formula (I) is:
2-(4-chloro-2-hydroxyphenyl)-1,3-benzothiazole-6-carboxylic acid;
2-(2-hydroxy-6-methoxyphenyl)-1,3-benzothiazole-6-carboxylic acid;
2-(2-hydroxy-5-methoxyphenyl)-1,3-benzothiazole-6-carboxylic acid;
2-(4-chloro-2-hydroxyphenyl)-N-[2-(dimethylamino)ethyl]-N-methyl-1,3-benzothiazole-6-carboxamide;
5-chloro-2-[6-(pyrrolidin-1-ylcarbonyl)-1,3-benzothiazol-2-yl]phenol;
2-(4-chloro-2-hydroxyphenyl)-N-(3-morpholin-4-ylpropyl)-1,3-benzothiazole-6-carboxamide;
2-(4-chloro-2-hydroxyphenyl)-N-(2-pyrrolidin-1-ylethyl)-1,3-benzothiazole-6-carboxamide;
5-chloro-2-(6-{[4-(2-methoxyethyl)piperazin-1-yl]carbonyl}-1,3-benzothiazol-2-yl)phenol;
2-(2-hydroxy-5-methoxyphenyl)-N-(3-morpholin-4-ylpropyl)-1,3-benzothiazole-6-carboxamide;
4-methoxy-2-[6-(pyrrolidin-1-ylcarbonyl)-1,3-benzothiazol-2-yl]phenol;
2-(5-bromo-2-hydroxyphenyl)-N-[2-(dimethylamino)ethyl]-N-methyl-1,3-benzothiazole-6-carboxamide;
4-fluoro-2-[6-(pyrrolidin-1-ylcarbonyl)-1,3-benzothiazol-2-yl]phenol;
5-methoxy-2-[6-(pyrrolidin-1-ylcarbonyl)-1,3-benzothiazol-2-yl]phenol;
methyl N- {[2-(2-hydroxyphenyl)-1,3-benzothiazol-6-yl]carbonyl}serinate;
2-[6-(pyrrolidin-1-ylcarbonyl)-1,3-benzothiazol-2-yl]phenol;
2-(5-chloro-2-hydroxyphenyl)-N-(3-morpholin-4-ylpropyl)-1,3-benzothiazole-6-carboxamide;
3-methoxy-2-[6-(pyrrolidin-1-ylcarbonyl)-1,3-benzothiazol-2-yl]phenol;
N,N-diethyl-2-(2-hydroxy-4-methoxyphenyl)-1,3-benzothiazole-6-carboxamide;
N-[2-hydroxy-1-(hydroxymethyl)ethyl]-2-(2-hydroxy-4-methoxyphenyl)-1,3-benzothiazole-6-carboxamide;
2-[6-(pyrrolidin-1-ylcarbonyl)-1, 3-benzothiazol-2-yl] pyridin-3-ol;
methyl 4-{[2-(6-{[[2-(dimethylamino)ethyl](methyl)amino]carbonyl}-1,3-benzothiazol-2-yl)-3-hydroxyphenoxy]methyl} benzoate;
5-ethoxy-4-[6-(pyrrolidin-1-ylcarbonyl)- 1,3-benzothiazol-2-yl]pyridin-3-ol;
N-[2-(dimethylamino)ethyl]-2-(4-hydroxy-1,1'-biphenyl-3-yl)-N-methyl-1,3-benzothiazole-6-carboxamide;
N-[2-(dimethylamino)ethyl]-2-(4-hydroxy-3'-methoxy-1,1'-biphenyl-3-yl)-N-methyl-1,3-benzothiazole-6-carboxamide;
5-chloro-2-(6- {[[2-(dimethylamino)ethyl](methyl)amino]methyl}-1,3-benzothiazol-2-yl)phenol;
5-chloro-2-(6-{[4-(2-methoxyethyl)piperazin-1-yl]methyl}-1,3-benzothiazol-2-yl)phenol;
5-chloro-2-[6-(pyrrolidin-1-ylmethyl)-1,3-benzothiazol-2-yl]phenol;
4-fluoro-2-[6-(pyrrolidin-1-ylmethyl)-1,3-benzothiazol-2-yl]phenol;
2-[6-(pyrrolidin-1-ylmethyl)-1,3-benzothiazol-2-yl]phenol;
3-methoxy-2-[6-(pyrrolidin-1-ylmethyl)-1,3-benzothiazol-2-yl]phenol;
4-methoxy-2-[6-(pyrrolidin-1-ylmethyl)-1,3-benzothiazol-2-yl]phenol;
4-bromo-2-[6-(pyrrolidin-1-ylmethyl)-1,3-benzothiazol-2-yl]phenol;
5-methoxy-2-[6-(pyrrolidin-1-ylmethyl)-1,3-benzothiazol-2-yl]phenol;
4-chloro-2-[6-(pyrrolidin-1-ylmethyl)-1,3-benzothiazol-2-yl]phenol;
2-{6-[(diethylamino)methyl]-1,3-benzothiazol-2-yl}-5-methoxyphenol;
5-ethoxy-4-[6-(pyrrolidin-1-ylmethyl)-1,3-benzothiazol-2-yl]pyridin-3-ol;
4-chloro-2-(6-{([(3-morpholin-4-ylpropyl)amino]methyl}-1,3-benzothiazol-2-yl)phenol;
2-[6-(pyrrolidin-1-ylmethyl)-1,3-benzothiazol-2-yl]pyridin-3-ol;
2-[5-(pyrrolidin-1-ylmethyl)-1,3-benzothiazol-2-yl]phenol;
5-chloro-2-[6-(piperidin-1-ylmethyl)-1,3-benzothiazol-2-yl]phenol;
5-chloro-2-[6-(4-methylpiperazin-1-ylmethyl)-1,3-benzothiazol-2-yl]phenol;
5-chloro-2- {6-[(diethylamino)methyl]-1,3-benzothiazol-2-yl}phenol;
5-chloro-2-{6-[(dimethylamino)methyl]-1,3-benzothiazol-2-yl}phenol;
2-[6-(hydroxymethyl)-1,3-benzothiazol-2-yl]phenol;
2-(6-amino-1,3-benzothiazol-2-yl)-4-methoxyphenol;
*N,N*-dimethyl-2-(2-hydroxy-4-methoxyphenyl)-1,3-benzothiazole-6-carboxamide;
*N,N-*dimethyl-2-(2-hydroxyphenyl)-1,3-benzothiazole-6-carboxamide;
3-[6-(pyrrolidin-1-ylcarbonyl)-1,3-benzothiazol-2-yl]pyrazin-2-amine;
2-(4-chloro-2-hydroxyphenyl)-1,3-benzothiazol-6-ol;
5-chloro-2-[6-(2-hydroxy-3-pyrrolidin-1-yl-propoxy)-1,3-benzothiazol-2-yl]-phenol;
5-chloro-2-[5-(pyrrolidin-1-ylcarbonyl)-1,3-benzothiazol-2-yl]phenol;
2-[5-(pyrrolidin-1-ylcarbonyl)-1,3-benzothiazol-2-yl]phenol;
*N,N*-dimethyl-2-(2-hydroxyphenyl)-1,3-benzothiazole-5-carboxamide;.
5-chloro-2-[5-(pyrrolidin-1-ylmethyl)-1,3-benzothiazol-2-yl]phenol;
2-{5-[(dimethylamino)methyl]-1,3-benzothiazol-2-yl}phenol;
2-(2-hydroxyphenyl)-N-(3-morpholin-4-ylpropyl)-1,3-benzothiazole-7-carboxamide;
or a pharmaceutically acceptable salt of any one thereof.

8. A compound of formula (Ia) 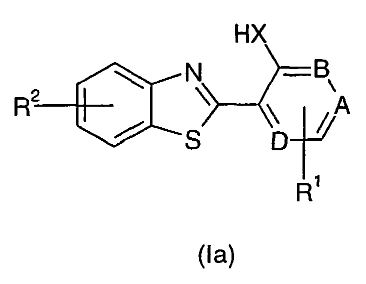 wherein:
A and D independently represent CH or N; and
either B represents CH and X represents O;
or B represents N and X represents NH;
R¹ represents hydrogen, halogen, C1 to 6 alkyl, C1 to 6 alkoxy, aryl or benzyloxy; said aryl or benzyloxy group being optionally further substituted by a group selected from C1 to 6 alkyl, C1 to 6 alkoxy, halogen and carbomethoxy;
R², which is attached to the 5- or 6-position of the benzothiazole ring, represents CONR³R⁴ or C1 to 6 alkyl or C1 to 6 alkoxy; said alkyl or alkoxy group being further substituted by one or more groups independently selected from hydroxy and NR⁵R⁶;
NR³R⁴ represents a 5 to 7 membered azacyclic ring optionally incorporating one further heteroatom selected from O and NR⁹;
R⁵ and R⁶ each independently represent hydrogen or C1 to 6 alkyl; said alkyl group being optionally further substituted by one or more substituents selected independently from hydroxy, C1 to 6 alkoxy, NR⁷R⁸ and C1 to 6 alkoxycarbonyl;
or the group NR⁵R⁶ together represents a 5 to 7 membered azacyclic ring optionally incorporating one further heteroatom selected from O and NR⁹;
R⁷ and R⁸ independently represent hydrogen or C1 to 6 alkyl; or the group NR⁷R⁸ together represents a 5 to 7 membered azacyclic ring optionally incorporating one further heteroatom selected from O and NR⁹;
R⁹ represents hydrogen or C1 to 6 alkyl; said alkyl group being optionally further substituted by C1 to 6 alkoxy;
or a pharmaceutically acceptable salts thereof.

9. A compound of formula (Ia), according to Claim 8, for use as a medicament.

10. A pharmaceutical formulation comprising a compound of formula (Ia), as defined in Claim 8, or a pharmaceutically acceptable salt thereof, optionally in admixture with a pharmaceutically acceptable diluent or carrier.

11. A process for the preparation of a compound of formula (Ia), as defined in Claim 8, and optical isomers, racemates and tautomers thereof and pharmaceutically acceptable salts thereof, which comprises:
(a) reaction of a compound of formula (II) 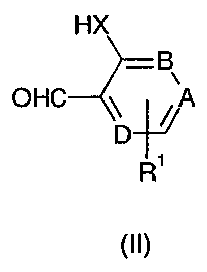 wherein R¹, A, B, D and X are as defined in Claim 8, with a compound of formula (III) 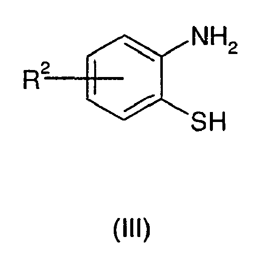 wherein R² is as defined in Claim 8; or
(b) oxidative cyclisation of a compound of formula (IV) 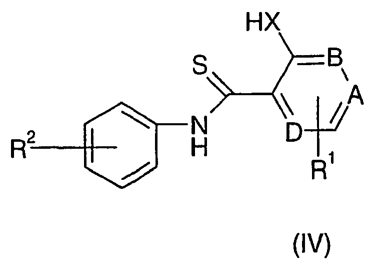 wherein R¹, R², A, B, D and X are as defined in Claim 8;
and where desired or necessary converting the resultant compound of formula (Ia), or another salt thereof, into a pharmaceutically acceptable salt thereof; or converting one compound of formula (Ia) into another compound of formula (Ia); and where desired converting the resultant compound of formula (Ia) into an optical isomer thereof.

## Patentansprüche

1. Verwendung einer Verbindung der Formel (I) 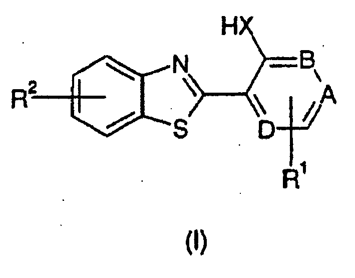 wobei:
A und D unabhängig voneinander für CH oder N stehen; und
entweder B für CH steht und X für O steht;
oder B für N steht und X für NH steht;
R¹ für Wasserstoff, Halogen, C₁₋₆-Alkyl, C₁₋₆-Alkoxy, Aryl oder Benzyloxy steht; wobei die Aryl- bzw. Benzyloxygruppe gegebenenfalls weiter substituiert ist durch eine Gruppe ausgewählt aus C₁₋₆-Alkyl, C₁-₆₋₋Alkoxy, Halogen und Carbomethoxy;
R² für Hydroxy, Amino, C₁₋₆-Alkyl, C₁₋₆-Alkoxy, Carbamoyl (-CONR³R⁴) oder -COOH steht; wobei die Alkyl- bzw. Alkoxygruppe gegebenenfalls weiter substituiert ist durch eine oder mehrere Gruppen unabhängig voneinander ausgewählt aus Hydroxy und NR⁵R⁶ ;
R³, R⁴, R⁵ und R⁶ jeweils unabhängig voneinander für Wasserstoff oder C₁₋₆-Alkyl stehen; wobei die Alkylgruppe gegebenenfalls weiter substituiert ist durch einen oder mehrere Substituenten unabhängig voneinander ausgewählt aus Hydroxy, C₁₋₆-Alkoxy, NR⁷R⁸ und C₁₋₆-Alkoxycarbonyl;
oder die Gruppe NR³R⁴ bzw. die Gruppe NR⁵R⁶ können zusammen für einen 5- bis 7gliedrigen azacyclischen Ring stehen, der gegebenenfalls ein weiteres Heteroatom ausgewählt aus O und NR⁹ enthält;
R⁷ und R⁸ unabhängig voneinander für Wasserstoff oder C₁₋₆-Alkyl stehen; oder die Gruppe NR⁷R⁸ zusammen für einen 5- bis 7gliedrigen azacyclischen Ring steht, der gegebenenfalls ein weiteres Heteroatom ausgewählt aus O und NR⁹ enthält;
R⁹ für Wasserstoff oder C₁₋₆-Alkyl steht; wobei die Alkylgruppe gegebenenfalls weiter substituiert ist durch C₁₋₆-Alkoxy;
oder eines pharmazeutisch annehmbaren Salzes davon bei der Herstellung eines Medikaments zur Behandlung oder Prophylaxe von Krankheiten bzw. Leiden, bei denen eine Inhibierung der Kinase-Itk-Aktivität von Nutzen ist.

2. Verwendung nach Anspruch 1 einer Verbindung der Formel (I) oder eines pharmazeutisch annehmbaren Salzes davon bei der Herstellung eines Medikaments zur Behandlung oder Prophylaxe von durch Th2 verursachten und/oder von Mastzellen verursachten und/oder von basophilen Zellen verursachten Krankheiten bzw. Leiden.

3. Verwendung nach Anspruch 2, wobei es sich bei der Krankheit um Asthma handelt.

4. Verwendung nach Anspruch 2, wobei es sich bei der Krankheit um allergische Rhinitis handelt.

5. Verwendung nach einem der Ansprüche 1 bis 4, wobei B in Formel (I) für CH steht und X für O steht.

6. Verwendung nach einem der Ansprüche 1 bis 5, wobei R² in Formel (I) für -CONR³R⁴ steht oder R² für durch NR⁵R⁶ substituiertes C₁₋₆-Alkyl steht.

7. Verwendung nach einem der Ansprüche 1 bis 4, wobei es sich bei der Verbindung der Formel (I) um:
2-(4-Chlor-2-hydroxyphenyl)-1,3-benzothiazol-6-carbonsäure;
2-(2-Hydroxy-6-methoxyphenyl)-1,3-benzothiazol-6-carbonsäure;
2-(2-Hydroxy-5-methoxyphenyl)-1,3-benzothiazol-6-carbonsäure;
2-(4-Chlor-2-hydroxyphenyl)-N-[2-(dimethylamino)ethyl]-N-methyl-1,3-benzothiazol-6-carbonsäureamid;
5-Chlor-2-[6-(pyrrolidin-1-ylcarbonyl)-1,3-benzothiazol-2-yl]phenol;
2-(4-Chlor-2-hydroxyphenyl)-N-(3-morpholin-4-ylpropyl)-1,3-benzothiazol-6-carbonsäureamid;
2-(4-Chlor-2-hydroxyphenyl)-N-(2-pyrrolidin-2-1-ylethyl)-1,3-benzothiazol-6-carbonsäureamid;
5-Chlor-2-(6-{[4-(2-methoxyethyl)piperazin-1-yl]carbonyl}-1,3-benzothiazol-2-yl)phenol;
2-(2-Hydroxy-5-methoxyphenyl)-N-(3-morpholin-4-ylpropyl)-1,3-benzothiazol-6-carbonsäureamid;
4-Methoxy-2-[6-(pyrrolidin-1-ylcarbonyl)-1,3-benzothiazol-2-yl]phenol;
2-(5-Brom-2-hydroxyphenyl)-N-[2-(dimethylamino)ethyl]-N-methyl-1,3-benzothiazol-6-carbonsäureamid;
4-Fluor-2-[6-(pyrrolidin-1-ylcarbonyl)-1,3-benzothiazol-2-yl]phenol;
5-Methoxy-2-[6-(pyrrolidin-1-ylcarbonyl)-1,3-benzothiazol-2-yl]phenol;
N-{[2-(2-Hydroxyphenyl)-1,3-benzothiazol-6-yl]carbonyl}serinmethylester;
2-[6-(Pyrrolidin-1-ylcarbonyl)-1,3-benzothiazol-2-yl]phenol;
2-(5-Chlor-2-hydroxyphenyl)-N-(3-morpholin-4-ylpropyl)-1,3-benzothiazol-6-carbonsäureamid;
3-Methoxy-2-[6-(pyrrolidin-1-ylcarbonyl)-1,3-benzothiazol-2-yl]phenol;
N,N-Diethyl-2-(2-hydroxy-4-methoxyphenyl)-1,3-benzothiazol-6-carbonsäureamid;
N-[2-Hydroxy-1-(hydroxymethyl)ethyl]-2-(2-hydroxy-4-methoxyphenyl)-1,3-benzothiazol-6-carbonsäureamid;
2-[6-(Pyrrolidin-1-ylcarbonyl)-1,3-benzothiazol-2-yl]pyridin-3-ol;
4-{[2-(6-{[[2-(Dimethylamino)ethyl](methyl)amino]carbonyl}-1,3-benzothiazol-2-yl)-3-hydroxyphenoxy]methyl}benzoesäuremethylester;
5-Ethoxy-4-[6-(pyrrolidin-1-ylcarbonyl)-1,3-benzothiazol-2-yl]pyridin-3-ol;
N-[2-(Dimethylamino)ethyl]-2-(4-hydroxy-1,1'-biphenyl-3-yl)-N-methyl-1,3-benzothiazol-6-carbonsäureamid;
N-[2-(Dimethylamino)ethyl]-2-(4-hydroxy-3'-methoxy-1,1'-biphenyl-3-yl)-N-methyl-1,3-benzothiazol-6-carbonsäureamid;
5-Chlor-2-(6-{[[2-(dimethylamino)ethyl](methyl)amino]methyl}-1,3-benzothiazol-2-yl)phenol;
5-Chlor-2-(6-{[4-(2-methoxyethyl)piperazin-1-yl]methyl}-1,3-benzothiazol-2-yl)phenol;
5-Chlor-2-[6-(pyrrolidin-1-ylmethyl)-1,3-benzothiazol-2-yl]phenol;
4-Fluor-2-[6-(pyrrolidin-1-ylmethyl)-1,3-benzothiazol-2-yl]phenol;
2-[6-(Pyrrolidin-1-ylmethyl)-1,3-benzothiazol-2-yl]phenol;
3-Methoxy-2-[6-(pyrrolidin-1-ylmethyl)-1,3-benzothiazol-2-yl]phenol;
4-Methoxy-2-[6-(pyrrolidin-1-ylmethyl)-1,3-benzothiazol-2-yl]phenol;
4-Brom-2-[6-(pyrrolidin-1-ylmethyl)-1,3-benzothiazol-2-yl]phenol;
5-Methoxy-2-[6-(pyrrolidin-1-ylmethyl)-1,3-benzothiazol-2-yl]phenol;
4-Chlor-2-[6-(pyrrolidin-1-ylmethyl)-1,3-benzothiazol-2-yl]phenol;
2-{6-[(Diethylamino)methyl]-1,3-benzothiazol-2-yl}-5-methoxyphenol;
5-Ethoxy-4-[6-(pyrrolidin-1-ylmethyl)-1,3-benzothiazol-2-yl]pyridin-3-ol;
4-Chlor-2-(6-{[(3-morpholin-4-ylpropyl)amino]methyl}-1,3-benzothiazol-2-yl)phenol;
2-[6-(Pyrrolidin-1-ylmethyl)-1,3-benzothiazol-2-yl]pyridin-3-ol;
2-[5-(Pyrrolidin-1-ylmethyl)-1,3-benzothiazol-2-yl]phenol;
5-Chlor-2-[6-(piperidin-1-ylmethyl)-1,3-benzothiazol-2-yl]phenol;
5-Chlor-2-[6-(4-methylpiperazin-1-ylmethyl)-1,3-benzothiazol-2-yl]phenol;
5-Chlor-2-{6-[(diethylamino)methyl]-1,3-benzothiazol-2-yl}phenol;
5-Chlor-2-{6-[(dimethylamino)methyl]-1,3-benzothiazol-2-yl}phenol;
2-[6-(Hydroxymethyl]-1,3-benzothiazol-2-yl]phenol;
2-(6-Amino-1,3-benzothiazol-2-yl)-4-methoxyphenol;
N,N-Dimethyl-2-(2-hydroxy-4-methoxyphenyl)-1,3-benzothiazol-6-carbonsäureamid;
N,N-Dimethyl-2-(2-hydroxyphenyl)-1,3-benzothiazol-6-carbonsäureamid;
3-[6-(Pyrrolidin-1-ylcarbonyl)-1,3-benzothiazol-2-yl]pyrazin-2-amin;
2-(4-(Chlor-2-hydroxyphenyl)-1,3-benzothiazol-6-ol;
5-Chlor-2-[6-(2-hydroxy-3-pyrrolidin-1-ylpropoxy)-1,3-benzothiazol-2-yl]phenol;
5-Chlor-2-[5-(pyrrolidin-1-ylcarbonyl)-1,3-benzothiazol-2-yl]phenol;
2-[5-(Pyrrolidin-1-ylcarbonyl)-1,3-benzothiazol-2-yl]phenol;
N,N-Dimethyl-2-(2-hydroxyphenyl)-1,3-benzothiazol-5-carbonsäureamid;
5-Chlor-2-[5-(pyrrolidin-1-ylmethyl)-1,3-benzothiazol-2-yl]phenol;
2-{5-[(Dimethylamino)methyl]-1,3-benzothiazol-2-yl}phenol;
2-(2-Hydroxyphenyl)-N-(3-morpholin-4-ylpropyl)-1,3-benzothiazol-7-carbonsäureamid;
oder ein pharmazeutisch annehmbares Salz einer dieser Verbindungen handelt.

8. Verbindungen der Formel (Ia) 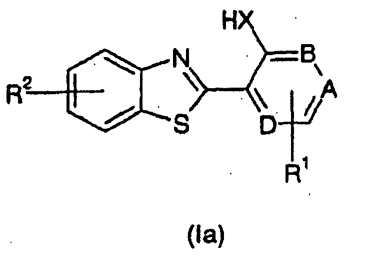 wobei:
A und D unabhängig voneinander für CH oder N stehen; und
entweder B für CH steht und X für O steht;
oder B für N steht und X für NH steht;
R¹ für Wasserstoff, Halogen, C₁₋₆-Alkyl, C₁₋₆-Alkoxy, Aryl oder Benzyloxy steht; wobei die Aryl- bzw. Benzyloxygruppe gegebenenfalls weiter substituiert ist durch eine Gruppe ausgewählt aus C₁₋₆-Alkyl, C₁₋₆₋Alkoxy, Halogen und Carbomethoxy;
R², das in der 5- oder 6-Stellung des Benzothiazolrings gebunden ist, für CONR³R⁴ oder C₁₋₆₋Alkyl oder C₁₋₆-Alkoxy steht; wobei die Alkyl- bzw. Alkoxygruppe weiter durch eine oder mehrere Gruppen unabhängig voneinander ausgewählt aus Hydroxy und NR⁵R⁶ substituiert ist;
NR³R⁴ für einen 5- bis 7gliedrigen azacyclischen Ring steht, der gegebenenfalls ein weiteres Heteroatom ausgewählt aus O und NR⁹ enthält;
R⁵ und R⁶ jeweils unabhängig voneinander für Wasserstoff oder C₁₋₆-Alkyl stehen; wobei die Alkylgruppe gegebenenfalls weiter substituiert ist durch einen oder mehrere Substituenten unabhängig voneinander ausgewählt aus Hydroxy, C₁₋₆-Alkoxy, NR⁷R⁸ und C₁₋₆-Alkoxycarbonyl;
oder die Gruppe NR⁵R⁶ zusammen für einen 5- bis 7gliedrigen azacyclischen Ring steht, der gegebenenfalls ein weiteres Heteroatom ausgewählt aus O und NR⁹ enthält;
R⁷ und R⁸ unabhängig voneinander für Wasserstoff oder C₁₋₆-Alkyl stehen; oder die Gruppe NR⁷R⁸ zusammen für einen 5- bis 7gliedrigen azacyclischen Ring steht, der gegebenenfalls ein weiteres Heteroatom ausgewählt aus O und NR⁹ enthält;
R⁹ für Wasserstoff oder C₁₋₆-Alkyl steht; wobei die Alkylgruppe gegebenenfalls weiter substituiert ist durch C₁₋₆-Alkoxy;
und deren pharmazeutisch annehmbare Salze.

9. Verbindungen der Formel (Ia) nach Anspruch 8 zur Verwendung als Medikament.

10. Pharmazeutische Formulierung, enthaltend eine wie in Anspruch 8 definierte Verbindung der Formel (Ia) oder ein pharmazeutisch annehmbares Salz davon, gegebenenfalls in einer Mischung mit einem pharmazeutisch annehmbaren Verdünnungsmittel oder Träger.

11. Verfahren zur Herstellung einer wie in Anspruch 8 definierten Verbindung der Formel (Ia) und optischen Isomeren, Racematen und Tautomeren davon und pharmazeutisch annehmbaren Salzen davon, bei dem man:
(a) eine Verbindung der Formel (II) 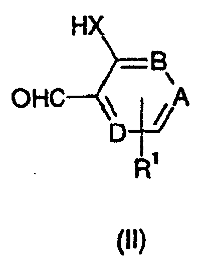 in welcher R¹, A, B, D und X wie in Anspruch 8 definiert sind, mit einer Verbindung der Formel (III) 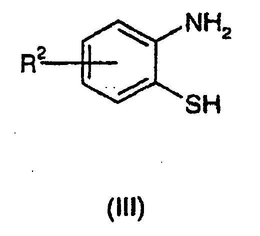 in welcher R² wie in Anspruch 8 definiert ist, umsetzt; oder
(b) eine Verbindung der Formel (IV) 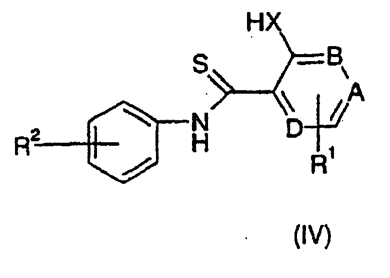 in welcher R¹, R², A, B, D und X wie in Anspruch 8 definiert sind, oxidativ cyclisiert;
und, falls gewünscht oder erforderlich, die so erhaltene Verbindung der Formel (Ia) oder ein anderes Salz davon in ein pharmazeutisch annehmbares Salz davon umwandelt; oder eine Verbindung der Formel (Ia) in eine andere Verbindung der Formel (Ia) umwandelt; und, falls gewünscht, die so erhaltene Verbindung der Formel (Ia) in ein optisches Isomer davon umwandelt.

## Revendications

1. Utilisation d'un composé de formule (I) 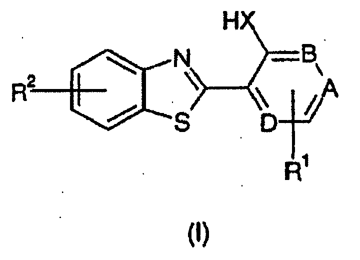 dans laquelle :
A et D représentent indépendamment CH ou N ; et
soit B représente CH et X représente O ;
soit B représente N et X représente NH ;
R¹ représente hydrogène, halogène, alkyle en C₁₋₆, alcoxy en C₁₋₆, aryle ou benzyloxy ; ledit groupement aryle ou benzyloxy étant éventuellement encore substitué par un groupement choisi parmi alkyle en C₁₋₆, alcoxy en C₁₋₆, halogène et carbométhoxy ;
R² représente hydroxy, amino, alkyle en C₁₋₆, alcoxy en C₁₋₆, carbamoyle (-CONR³R⁴) ou -COOH ; ledit groupement alkyle ou alcoxy étant éventuellement encore substitué par un ou plusieurs groupements choisis indépendamment parmi hydroxy et NR⁵R⁶;
R³, R⁴, R⁵ et R⁶ représentent chacun indépendamment hydrogène ou alkyle en C₁₋₆ ; ledit groupement alkyle étant éventuellement encore substitué par un ou plusieurs substituants choisis indépendamment parmi hydroxy, alcoxy en C₁₋₆, NR⁷R⁸ et alcoxycarbonyle en C₁₋₆ ;
ou le groupement NR³R⁴ ou le groupement NR⁵R⁶ peuvent représenter ensemble un cycle azacyclique de 5 à 7 chaînons comportant éventuellement un hétéroatome supplémentaire choisi parmi O et NR⁹ ;
R⁷ et R⁸ représentent indépendamment hydrogène ou alkyle en C₁₋₆ ; ou le groupement NR⁷R⁸ représente ensemble un cycle azacyclique de 5 à 7 chaînons comportant éventuellement un hétéroatome supplémentaire choisi parmi O et NR⁹ ;
R⁹ représente hydrogène ou alkyle en C₁₋₆ ; ledit groupement alkyle étant éventuellement encore substitué par alcoxy en C₁₋₆ ;
ou un sel pharmaceutiquement acceptable de celui-ci, dans la fabrication d'un médicament destiné au traitement ou à la prophylaxie de maladies ou d'affections dans lesquelles l'inhibition de l'activité de la kinase Itk est bénéfique.

2. Utilisation selon la revendication 1, d'un composé de formule (I) ou d'un sel pharmaceutiquement acceptable de celui-ci, dans la fabrication d'un médicament destiné au traitement ou à la prophylaxie de maladies ou d'affections favorisées par Th2 et/ou favorisées par des mastocytes et/ou favorisées par des basophiles.

3. Utilisation selon la revendication 2, **caractérisée en ce que** la maladie est l'asthme.

4. Utilisation selon la revendication 2, **caractérisée en ce que** la maladie est la rhinite allergique.

5. Utilisation selon l'une quelconque des revendications 1 à 4, **caractérisée en ce que** B dans la formule (I) représente CH et X représente O.

6. Utilisation selon l'une quelconque des revendications 1 à 5, **caractérisée en ce que** R² dans la formule (I) représente -CONR³R⁴ ou R² représente alkyle en C₁₋₆ substitué par NR⁵R⁶.

7. Utilisation selon l'une quelconque des revendications 1 à 4, **caractérisée en ce que** le composé de formule (I) est :
l'acide 2-(4-chloro-2-hydroxyphényl)-1,3-benzothiazole-6-carboxylique ;
l'acide 2-(2-hydroxy-6-méthoxyphényl)-1,3-benzothiazole-6-carboxylique ;
l'acide 2-(2-hydroxy-5-méthoxyphényl)-1,3-benzothiazole-6-carboxylique ;
le 2-(4-chloro-hydroxyphényl)-N-[2-(diméthylamino)éthyl] N-méthyl-1,3-benzothiazole-6-carboxamide ;
le 5-chloro-2-[6-(pyrrolidin-1-ylcarbonyl)-1,3-benzothiazol-2-yl]phénol ;
le 2-(4-chloro-2-hydroxyphényl)-N-(3-morpholin-4-ylpropyl)-1,3-benzothiazole-6-carboxamide ;
le 2-(4-chloro-2-hydroxyphényl)-N-(2-pyrrolidin-1-yléthyl)-1,3-benzothiazole-6-carboxamide ;
le 5-chloro-2-(6-([4-(2-méthoxyéthyl)pipérazin-1-yl]carbonyl)-1,3-benzothiazole-2-yl)phénol ;
le 2-(2-hydroxy-5-méthoxyphényl)-N-(3-morpholin-4-ylpropyl)-1,3-benzothiazole-6-carboxamide ;
le 4-méthoxy-2-[6-(pyrrolidin-1-ylcarbonyl)-1,3-benzothiazol-2-yl]phénol ;
le 2-(5-bromo-2-hydroxyphényl)-N-[2-(diméthylamino)éthyl]-N-méthyl-1,3-benzothiazole-6-carboxamide ;
le 4-fluoro-2-[6-(pyrrolidin-1-ylcarbonyl)-1,3-benzothiazol-2-yl]phénol ;
le 5-méthoxy-2-[6-(pyrrolidin-1-ylcarbonyl)-1,3-benzothiazol-2-yl]phénol ;
le N-{[2-(2-hydroxyphényl)-1,3-benzothiazol-6-yl]carbonyl}sérinate de méthyle ;
le 2-[6-(pyrrolidin-1-ylcarbonyl)-1,3-benzothiazol-2-yl]phénol ;
le 2-(5-chloro-2-hydroxyphényl)-N-(3-morpholin-4-ylpropyl)-1,3-benzothiazole-6-carboxamide ;
le 3-méthoxy-2-[6-(pyrrolidin-1-ylcarbonyl)-1,3-benzothiazol-2-yl]phénol ;
le N,N-diéthyl-2-(2-hydroxy-4-méthoxyphényl)-1,3-benzothiazole-6-carboxamide ;
le N-[2-hydroxy-1-(hydroxyméthyl)éthyl]-2-(2-hydroxy-4-méthoxyphényl)-1,3-benzothiazole-6-carboxamide ;
le 2-[6-(pyrrolidin-1-ylcarbonyl)-1,3-benzothiazol-2-yl]pyridin-3-ol ;
le 4-{[2-(6-{[[2-(diméthylamino)éthyl]-(méthyl)amino]carbonyl}-1,3-benzothiazol-2-yl)-3-hydroxyphénoxy]méthyl}benzoate de méthyle;
le 5-éthoxy-4-[6-(pyrrolidin-1-ylcarbonyl)-1,3-benzothiazol-2-yl]pyridin-3-ol ;
le N-[2-(diméthylamino)éthyl]-2-(4-hydroxy-1,1'-biphényl-3-yl)-N-méthyl-1,3-benzothiazole-6-carboxamide ;
le N-[2-(diméthylamino)éthyl]-2-(4-hydroxy-3'-méthoxy-1,1'-biphényl-3-yl)-N-méthyl-1,3-benzothiazole-6-carboxamide ;
le 5-chloro-2-(6-{[[2-(diméthylamino)éthyl]-(méthyl)amino]méthyl}-1,3-benzothiazol-2-yl)phénol ;
le 5-chloro-2-(6-{[4-(2-méthoxyéthyl)pipérazin-1-yl]méthyl}-1,3-benzothiazol-2-yl)phénol ;
le 5-chloro-2-[6-(pyrrolidin-1-ylméthyl)-1,3-benzothiazol-2-yl]phénol ;
le 4-fluoro-2-[6-(pyrrolidin-1-ylméthyl)-1,3-benzothiazol-2-yl]phénol ;
le 2-[6-(pyrrolidin-1-ylméthyl)-1,3-benzothiazol-2-yl]phénol;
le 3-méthoxy-2-[6-(pyrrolidin-1-ylméthyl)-1,3-benzothiazol-2-yl]phénol ;
le 4-méthoxy-2-[6-(pyrrolidin-1-ylméthyl)-1,3-benzothiazol-2-yl]phénol ;
le 4-bromo-2-[6-(pyrrolidin-1-ylméthyl)-1,3-benzothiazol-2-yl]phénol ;
le 5-méthoxy-2-[6-(pyrrolidin-1-ylméthyl)-1,3-benzothiazol-2-yl]phénol ;
le 4-chloro-2-[6-(pyrrolidin-1-ylméthyl)-1,3-benzothiazol-2-yl]phénol ;
le 2-{6-[(diéthylamino)méthyl]-1,3-benzothiazol-2-yl}-5-méthoxyphénol ;
le 5-éthoxy-4-[6-(pyrrolidin-1-ylméthyl)-1,3-benzothiazol-2-yl]pyridin-3-ol ;
le 4-chloro-2-(6-{[(3-morpholin-4-ylpropyl)amino]-méthyl}-1,3-benzothiazol-2-yl)phénol ;
le 2-[6-(pyrrolidin-1-ylméthyl)-1,3-benzothiazol-2-yl]pyridin-3-ol ;
le 2-[5-(pyrrolidin-1-ylméthyl)-1,3-benzothiazol-2-yl]phénol ;
le 5-chloro-2-[6-(pipéridin-1-ylméthyl)-1,3-benzothiazol-2-yl]phénol ;
le 5-chloro-2-[6-(4-méthylpipérazin-1-ylméthyl)-1,3-benzothiazol-2-yl]phénol ;
le 5-chloro-2-{6-[(diéthylamino)méthyl]-1,3-benzothiazol-2-yl}phénol ;
le 5-chloro-2-{6-[(diméthylamino)méthyl]-1,3-benzothiazol-2-yl}phénol ;
le 2-[6-(hydroxyméthyl)1,3-benzothiazol-2-yl]-phénol ;
le 2-(6-amino-1,3-benzothiazol-2-yl)-4-méthoxyphénol ;
le N,N-diméthyl-2-(2-hydroxy-4-méthoxyphényl)-1,3-benzothiazole-6-carboxamide ;
le N,N-diméthyl-2-(2-hydroxyphényl)-1,3-benzothiazole-6-carboxamide ;
la 3-[6-(pyrrolidin-1-ylcarbonyl)-1,3-benzothiazol-2-yl]pyrazin-2-amine ;
le 2-(4-chloro-2-hydroxyphényl)-1,3-benzothiazol-6-ol ;
le 5-chloro-2-[6-(2-hydroxy-3-pyrrolidin-1-yl-propoxy)-1,3-benzothiazol-2-yl]phénol ;
le 5-chloro-2-[5-(pyrrolidin-1-ylcarbonyl)-1,3-benzothiazol-2-yl]phénol ;
le 2-[5-(pyrrolidin-1-ylcarbonyl)-1,3-benzothiazol-2-yl]phénol ;
le N,N-diméthyl-2-(2-hydroxyphényl)-1,3-benzothiazole-5-carboxamide ;
le 5-chloro-2-[5-(pyrrolidin-1-ylméthyl)-1,3-benzothiazol-2-yl]phénol ;
le 2-{5-[(diméthylamino)méthyl]-1,3-benzothiazol-2-yl}phénol ;
le 2-(2-hydroxyphényl)-N-(3-morpholin-4-ylpropyl)-1,3-benzothiazole-7-carboxamide ;
ou un sel pharmaceutiquement acceptable de l'un quelconque de ceux-ci.

8. Composé de formule (Ia) 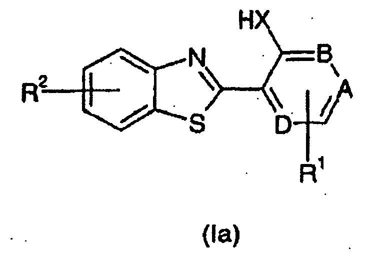 dans laquelle:
A et D représentent indépendamment CH ou N; et
soit B représente CH et X représente O ;
soit B représente N et X représente NH ;
R¹ représente hydrogène, halogène, alkyle en C₁₋₆, alcoxy en C₁₋₆, aryle ou benzyloxy ; ledit groupement aryle ou benzyloxy étant éventuellement encore substitué par un groupement choisi parmi alkyle en C₁₋₆, alcoxy en C₁₋₆, halogène et carbométhoxy;
R², qui est relié à la position 5 ou 6 du cycle benzothiazole, représente CONR³R⁴ ou alkyle en C₁₋₆ ou alcoxy en C₁₋₆; ledit groupement alkyle ou alcoxy étant encore substitué par un ou plusieurs groupements choisis indépendamment parmi hydroxy et NR⁵R⁶ ;
NR³R⁴ représente un cycle azacyclique de 5 à 7 chaînons comportant éventuellement un hétéroatome supplémentaire choisi parmi O et NR⁹ ;
R⁵ et R⁶ représentent chacun indépendamment hydrogène ou alkyle en C₁₋₆ ; ledit groupement alkyle étant éventuellement encore substitué par un ou plusieurs substituants choisis indépendamment parmi hydroxy, alcoxy en C₁₋₆, NR⁷R⁸ et alcoxycarbonyle en C₁₋₆ ;
ou le groupement NR⁵R⁶ représente ensemble un cycle azacyclique de 5 à 7 chaînons comportant éventuellement un hétéroatome supplémentaire choisi parmi O et NR⁹ ;
R⁷ et R⁸ représentent indépendamment hydrogène ou alkyle en C₁₋₆ ; ou le groupement NR⁷R⁸ représente ensemble un cycle azacyclique de 5 à 7 chaînons comportant éventuellement un hétéroatome supplémenaire choisi parmi O et NR⁹ ;
R⁹ représente hydrogène ou alkyle en C₁₋₆ ; ledit groupement alkyle étant éventuellement encore substitué par alcoxy en C₁₋₆ ;
ou un sel pharmaceutiquement acceptable de celui-ci.

9. Composé de formule (Ia) selon la revendication 8, destiné à être utilisé comme médicament.

10. Formulation pharmaceutique comprenant un composé de formule (Ia), tel que défini dans la revendication 8, ou un sel pharmaceutiquement acceptable de celui-ci, éventuellement en mélange avec un diluant ou un support pharmaceutiquement acceptable.

11. Procédé de préparation d'un composé de formule (Ia), tel que défini dans la revendication 8, et des isomères optiques, des racémates et des tautomères de celui-ci, et des sels pharmaceutiquement acceptables de celui-ci, qui comprend :
(a) la réaction d'un composé de formule (II) 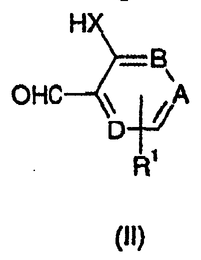 dans laquelle R¹, A, B, D et X sont tels que définis dans la revendication 8, avec un composé de formule (III) 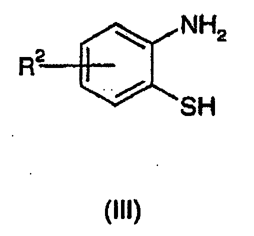 dans laquelle R² est tel que défini dans la revendication 8 ; ou
(b) la cyclisation oxydative d'un composé de formule (IV) 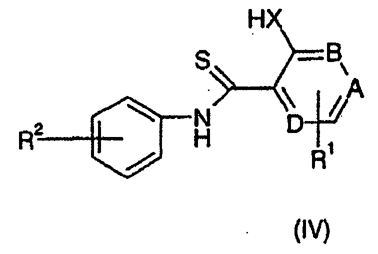 dans laquelle R¹, R², A, B, D et X sont tels que définis dans la revendication 8 ;
et si on le souhaite ou si nécessaire, la transformation du composé résultant de formule (Ia), ou d'un autre sel de celui-ci, en un sel pharmaceutiquement acceptable de celui-ci ; ou la transformation d'un composé de formule (Ia) en un autre composé de formule (Ia) ; et si on le souhaite la transformation du composé résultant de formule (Ia) en un isomère optique de celui-ci.
